(19) Europäisches Patentamt
European Patent Office
Office européen des brevets

(11) **EP 4 621 041 A1**

(12) **EUROPEAN PATENT APPLICATION**
published in accordance with Art. 153(4) EPC

(43) Date of publication:
**24.09.2025 Bulletin 2025/39**

(21) Application number: **23891482.4**

(22) Date of filing: **10.11.2023**

(51) International Patent Classification (IPC):
*C12M 1/34* (2006.01)      *C12M 1/00* (2006.01)
*C12M 3/00* (2006.01)      *C12N 1/00* (2006.01)
*C12N 1/02* (2006.01)      *C12Q 1/02* (2006.01)
*G01N 37/00* (2006.01)

(52) Cooperative Patent Classification (CPC):
**C12M 1/00; C12M 1/34; C12M 3/00; C12N 1/00;
C12N 1/02; C12Q 1/02; G01N 37/00**

(86) International application number:
**PCT/JP2023/040557**

(87) International publication number:
**WO 2024/106334 (23.05.2024 Gazette 2024/21)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **14.11.2022 JP 2022181803**

(71) Applicant: **TOKYO OHKA KOGYO CO., LTD.
Kawasaki-shi, Kanagawa 211 0012 (JP)**

(72) Inventors:
• **OHSAKA, Takashi**
  **Kawasaki-shi, Kanagawa 211-0012 (JP)**
• **ASAI, Takahiro**
  **Kawasaki-shi, Kanagawa 211-0012 (JP)**
• **TAKAHASHI, Anna**
  **Kawasaki-shi, Kanagawa 211-0012 (JP)**
• **NAKAMURA, Akimasa**
  **Kawasaki-shi, Kanagawa 211-0012 (JP)**

(74) Representative: **Plasseraud IP
104 Rue de Richelieu
CS92104
75080 Paris Cedex 02 (FR)**

(54) **DEVICE, OBSERVATION METHOD, RECOVERY METHOD, CULTURE METHOD, AND DEVICE MANUFACTURING METHOD**

(57)      A device (1) is a device including a plurality of units (3) of which each includes a sample chamber (2) that is able to individually accommodate cells or particles. Each unit (3) includes an accommodation layer (5) including an accommodation portion (4) corresponding to the sample chamber (2) and a structure (6) allowing a liquid to flow into the accommodation portion (4). The plurality of units (3) are independent there is no flow of the liquid therebetween.

FIG. 3

EP 4 621 041 A1

**Description**

TECHNICAL FIELD

The present invention relates to a device, an observation method, a recovery method, a culture method, and a device manufacturing method.

Priority is claimed on Japanese Patent Application No. 2022-181803, filed November 14, 2022, the content of which is incorporated herein by reference.

BACKGROUND ART

For example, a cell screening device that screens cells has been disclosed in Patent Document 1. The cell screening device includes a bottom plate portion, a cell mounting film that is provided on the bottom plate portion and constitutes a cell mounting surface, a pair of fluid injection portions that is provided to be partitioned from the cell mounting surface on the bottom plate portion, and a flow passage that is provided between the bottom plate portion and the cell mounting surface and of which a flow passage end extends to the fluid injection portions. In the cell mounting surface of the cell mounting film, a plurality of wells with a size in which cells to be screened are individually accommodated and through-holes extending from an inner bottom surface of each well to communicate with the flow passage are formed. In this device, a sample chamber including the cell mounting film and the flow passage are formed by mechanical fitting.

Citation List

Patent Document

Patent Document 1: PCT International Publication No. WO 2021/065771

SUMMARY OF INVENTION

Technical Problem

However, when a sample chamber and a flow passage are formed by mechanical fitting, it is difficult to provide a plurality of sample chambers that can independently handle fluids. Accordingly, there is a high likelihood that a plurality of samples (cells or particles) cannot be observed in one device.

Therefore, an objective of the present invention is to independently observe a plurality of samples (cells or particles) in one device.

Solution to Problem

(1) A device according to an aspect of the present invention is a device including a plurality of units of which each includes a sample chamber that is able to individually accommodate cells or particles, wherein each unit includes an accommodation layer including an accommodation portion corresponding to the sample chamber and a structure allowing a liquid to flow into the accommodation portion, and wherein the plurality of units are independent such that there is no flow of the liquid therebetween.

With this configuration, since the accommodation portions of the units are independent, a plurality of independent sample chambers can be provided in one device. Accordingly, it is possible to independently observe a plurality of samples (cells or particles) in one device. For example, it is possible to perform experiment under a plurality of independent conditions in one device.

(2) In the device according to (1), the structure may include: a liquid-passing member including a liquid-passing port for allowing the liquid to flow to the accommodation portion and having the accommodation layer attached thereto; and a flow passage forming member attached to the liquid-passing member with the accommodation layer interposed therebetween and allowing the liquid to flow between the sample chamber, the accommodation layer, and the liquid-passing port.

The structure may include a liquid-passing member including a liquid-passing port for allowing the liquid to flow to the accommodation portion and having the accommodation layer attached thereto. The structure may include a flow passage forming member attached to the liquid-passing member with the accommodation layer interposed therebetween and allowing the liquid to flow between the sample chamber, the accommodation layer, and the liquid-passing port.

With this configuration, it is possible to construct the device using three members (the liquid-passing member, the accommodation layer, and the flow passage forming member).

(3) In the device according to (2), the liquid-passing member may include: a first member including the sample chamber and having the accommodation layer attached thereto; and a second member including the liquid-passing port and assembled into the first member.

The liquid-passing member may include a first member including the sample chamber and having the accommodation layer attached thereto. The liquid-passing member may include a second member including the liquid-passing port and assembled into the first member.

With this configuration, since the liquid-passing member is divided into two members (the first member and the second member), only the first member is attached to the accommodation layer. Since the first member is smaller than the second member, the accommodation layers can be formed on a substrate with a higher density when a plurality of accommodation layers corresponding to the first member are formed on one substrate and this is attached to the first member than when the device is constituted using only the three members (the single liquid-passing member, the accommodation layer, and the flow passage forming member). Accordingly, it is possible to greatly increase the number of accommodation layers provided per substrate with the same area.

(4) In the device according to (2) or (3), the flow passage forming member may include: a first sheet that is transparent; and a second sheet that is stacked on the first sheet and includes a communication hole connecting the liquid-passing port to the sample chamber.

The flow passage forming member may include a first sheet that is transparent. The flow passage forming member may include a second sheet that is stacked on the first sheet and includes a communication hole connecting the liquid-passing port to the sample chamber.

With this configuration, it is possible to obtain the flow passage forming member when one sample chamber is provided in one unit. For example, an adhesive layer may be provided on one side of each of the first sheet and the second sheet. Alternatively, an adhesive layer may be provided on both sides of the second sheet.

(5) In the device according to (2) or (3), the flow passage forming member may include: a first sheet that is transparent; a second sheet that is stacked on the first sheet and includes a communication hole connecting the liquid-passing port to the sample chamber; and a third sheet that is stacked on the second sheet and includes an opening hole at positions of the liquid-passing member corresponding to the liquid-passing port and the sample chamber.

The flow passage forming member may include a third sheet that is stacked on the second sheet and includes an opening hole at positions of the liquid-passing member corresponding to the liquid-passing port and the sample chamber.

With this configuration, it is possible to obtain the flow passage forming member when a plurality of sample chambers are provided in one unit. For example, it is possible to cause the sheets to adhere by providing an adhesive layer between the sheets. There are three following combinations associated with in what sheet an adhesive layer is to be provided.

(A) An adhesive layer may be provided on one side of each of the first sheet, the second sheet, and the third sheet.
(B) An adhesive layer may be provided on both sides of the second sheet and one side of the third sheet.
(C) An adhesive layer may be provided on one side of the second sheet and both sides of the third sheet.

(6) In the device according to (2) or (3), the flow passage forming member may include: a first sheet that is transparent; a second sheet that is stacked on the first sheet and includes a communication hole connecting the liquid-passing port to the sample chamber; a third sheet that is stacked on the second sheet and includes an opening hole at positions of the liquid-passing member corresponding to the liquid-passing port and the sample chamber; and a fourth sheet that is stacked on the third sheet and includes the same shape in a plan view as the third sheet, and an adhesive layer may be provided on both sides of the second sheet and both sides of the fourth sheet.

The flow passage forming member may include a fourth sheet that is stacked on the third sheet and includes the same shape in a plan view as the third sheet. An adhesive layer may be provided on both sides of the second sheet. An adhesive layer may be provided on both sides of the fourth sheet.

With this configuration, since an adhesive layer may not be provided on both sides of the third sheet, it is possible to reduce a contact between the adhesive layer of the third sheet and the flowing liquid as much as possible. For example, since an adhesive layer may not be provided on both sides of the third sheet by employing combinations (A) and (B) for providing the adhesive layer, it is possible to achieve this advantageous effect.

For example, when a flow passage is formed using the first sheet that is transparent and does not include an adhesive layer and a sheet including an adhesive layer on both sides thereof, the fourth sheet not including an adhesive layer and having the same shape in a plan view as the third sheet can be inserted between the second sheet and the third sheet.

(7) In the device according to any one of (2) to (6), the thickness of the flow passage forming member may be equal to or less than 2 mm.

The thickness of the flow passage forming member is preferably equal to or less than 1.5 mm, more preferably equal to or less than 1.0 mm, and still more preferably equal to or less than 0.5 mm.

With this configuration, it is possible to better facilitate observation with high magnification in comparison with a case in which the thickness of the flow passage forming member is greater than 2 mm.

(8) In the device according to any one of (1) to (7), the accommodation layer may include: a well array filter including a well opening of a size through which the cell or the particle is able to pass; and a well bottom corresponding to the well opening, and the well bottom may include a through-hole through which only the liquid is able to pass.

The accommodation layer may include a well opening of a size through which the cell or the particle is able to pass. The accommodation layer may include a well bottom corresponding to the well opening. The well bottom may include a through-hole through which only the liquid is able to pass.

With this configuration, it is possible to observe a plurality of samples (cells or particles) in one device including a well array filter.

(9) In the device according to any one of (1) to (8), the accommodation layer may be formed of a negative resist.

The accommodation layer may be formed of a positive resist.

With this configuration, it is possible to manufacture the accommodation layer using photolithography (for example, a photoresist application step, an exposure step, and a developing step) which is suitable for micromachining.

(10) In the device according to any one of (1) to (9), the device may have an outer shape of the same size as slide glass in a plan view, and two, four, six, or eight sample chambers may be provided in the device.

The device may have an outer shape of the same size as slide glass in a plan view. Two, four, six, or eight sample chambers may be provided in the device.

With this configuration, it is possible to obtain a device of the same size as slide glass in a plan view.

(11) In the device according to any one of (1) to (9), the device may have the same rectangular outer shape as a microplate in a plan view, A sample chambers may be provided in a short-side direction of the device, B sample chambers may be provided in a long-side direction of the device, and a ratio A:B of the number of sample chambers A arranged in the short-side direction in the device to the number of sample chambers B arranged in the long-side direction may be 2:3, 2:1.5, or 2:1.

The device may have the same rectangular outer shape as a microplate in a plan view. A sample chambers may be provided in a short-side direction of the device. B sample chambers may be provided in a long-side direction of the device. A ratio A:B of the number of sample chambers A arranged in the short-side direction in the device to the number of sample chambers B arranged in the long-side direction may be 2:3, 2:1.5, or 2:1.

With this configuration, when the ratio A:B is 2:3, the sample chambers can be provided at the same positions as wells of a microplate which can be commercially available. For example, similarly to the wells of the microplate, a patch between the sample chambers has only to be the same vertically and horizontally. When the ratio A:B is 2:1.5, an arrangement of one liquid-passing port + one sample chamber (when 48 sample chambers are provided in a 96 microplate) can be provided at the same positions as two neighboring wells in the microplate. When the ratio A:B is 2:1, an arrangement of two liquid-passing ports + one sample chamber (when 32 sample chambers are provided in a 96 microplate) can be provided at the same positions as three wells adjacent to each other vertically or horizontally. For example, a sample chamber and two liquid-passing ports are preferably provided such that the sample chamber is interposed between two liquid-passing ports.

(12) In the device according to any one of (1) to (9), the device may have an outer shape of the same size as a microplate in a plan view, and 32 sample chambers or 48 sample chambers may be provided in the device.

The device may have an outer shape of the same size as a microplate in a plan view. 32 sample chambers or 48 sample chambers may be provided in the device.

With this configuration, it is possible to obtain a device of the same size as a microplate in a plan view.

(13) An observation method according to an aspect of the present invention is an observation method of individually observing the cells or the particles using the device according to any one of (1) to (12) and a microscope.

The observation method may use the device according to any one of (1) to (12) and a microscope. The observation method may be an observation method of individually observing the cells or the particles using a microscope.

With this method, since the device is used, it is possible to independently observe a plurality of samples (cells or particles) in one device.

(14) A recovery method according to an aspect of the present invention is a recovery method of individually recovering the cells or the particles using the device according to any one of (1) to (12).

The recovery method may use the device according to any one of (1) to (12). The recovery method may be a recovery method of individually recovering the cells or the particles.

With this method, since the device is used, it is possible to independently recover a plurality of samples (cells or particles) in one device.

(15) A culture method according to an aspect of the present invention is a culture method of culturing the cells using the device according to any one of (1) to (12).

The culture method may use the device according to any one of (1) to (12). The culture method may be a culture method of culturing the cells.

With this method, since the device is used, it is possible to independently culture a plurality of samples (cells or particles) in one device.

(16) A device manufacturing method according to an aspect of the present invention is a device manufacturing method of manufacturing the device according to any one of (1) to (12), the device manufacturing method including: a first step of attaching the accommodation layer to a liquid-passing member including a liquid-passing port for allowing the liquid to flow to the accommodation portion; and a second step of attaching a flow passage forming member for allowing the liquid to flow between the sample chamber, the accommodation layer, and the liquid-passing port to the liquid-passing member with the accommodation layer interposed therebetween after the first step.

The device manufacturing method may be a device manufacturing method of manufacturing the device according to any one of (1) to (12). The device manufacturing method may include: a first step of attaching the accommodation layer to a liquid-passing member including a liquid-passing port for allowing the liquid to flow to the accommodation portion. The device manufacturing method may include a second step of attaching a flow passage forming member for allowing the liquid to flow between the sample chamber, the accommodation layer, and the liquid-passing port to the liquid-passing member with the accommodation layer interposed therebetween after the first step.

With this method, it is possible to obtain a device including three members (the liquid-passing member, the accommodation layer, and the flow passage forming member) through two steps (the first step and the second step).

(17) In the device manufacturing method according to (16), the first step may include: an attachment step of attaching the accommodation layer to a first member including the sample chamber; and an assembly step of manufacturing the liquid-passing member by assembling a second member including the liquid-passing port to the first member after the attachment step.

The first step may include an attachment step of attaching the accommodation layer to a first member including the sample chamber. The first step may include an assembly step of manufacturing the liquid-passing member by assembling a second member including the liquid-passing port to the first member after the attachment step.

With this method, since the liquid-passing member is divided into two members (the first member and the second member), the accommodation layer is attached to only the first member in the attachment step. Accordingly, when a member in which individual accommodation layers are manufactured on different substrates or a connected member in which individual accommodation layers are connected is used, it is possible to use a member in which accommodation layers are integrated with a high density in comparison with a case in which two steps (the first step and the second step) according to (16) are simply used. As a result, it is possible to greatly increase the number of substrates or connected members provided per unit area.

Advantageous Effects of Invention

With the device, the observation method, the recovery method, the culture method, and the device manufacturing method according to the aspects, it is possible to independently observe a plurality of samples (cells or particles) in one device.

BRIEF DESCRIPTION OF DRAWINGS

[FIG. 1] A perspective view of a device according to a first embodiment.
[FIG. 2] A diagram illustrating an example of high-magnification observation of a unit according to the first embodiment.
[FIG. 3] A diagram illustrating an example of a plurality of independent sample chambers according to the first embodiment.
[FIG. 4] An exploded view of a unit according to the first embodiment.
[FIG. 5] A top view of a first member according to the first embodiment.
[FIG. 6] A diagram illustrating an accommodation layer according to the first embodiment along with a cross-section taken along line VI-VI in FIG. 5.
[FIG. 7] A bottom view of the accommodation layer according to the first embodiment.
[FIG. 8] A bottom view of the first member according to the first embodiment.
[FIG. 9] A diagram illustrating a capillary along with a partially enlarged view of FIG. 6.
[FIG. 10] A diagram illustrating the accommodation layer (an example of a well array filter) according to the first embodiment.

[FIG. 11] A top view of a second member according to the first embodiment.

[FIG. 12] A sectional view taken along line XII-XII in FIG. 11.

[FIG. 13] A diagram illustrating the first member along with the sectional view of FIG. 12.

[FIG. 14] A sectional view taken along line XIV-XIV in FIG. 11.

[FIG. 15] A sectional view taken along line XV-XV in FIG. 11.

[FIG. 16] A diagram illustrating the first member along with the sectional view of FIG. 15.

[FIG. 17] A bottom view of the second member according to the first embodiment.

[FIG. 18] A diagram illustrating a configuration of a flow passage forming member according to the first embodiment, where FIG. 18(A) is a top view of a first sheet, FIG. 18(B) is a top view of a second sheet, FIG. 18(C) is a top view of a third sheet, and FIG. 18(D) is a top view of a fourth sheet.

[FIG. 19] A sectional view of the flow passage forming member in the cross-section taken along XIX-XIX in FIG. 18.

[FIG. 20] A sectional view of the flow passage forming member in the cross-section taken along XX-XX in FIG. 18.

[FIG. 21] A diagram illustrating an example of a flow of a liquid in the flow passage forming member according to the first embodiment.

[FIG. 22] A diagram illustrating a shape of through-holes in the flow passage forming member according to the first embodiment.

[FIG. 23] A diagram illustrating a 96 microplate in which 32 sample chambers are provided.

[FIG. 24] An exploded view of a unit according to a second embodiment.

[FIG. 25] A top view of a unit according to a third embodiment.

[FIG. 26] An exploded top view of the unit according to the third embodiment.

[FIG. 27] A side view of the unit according to the third embodiment.

[FIG. 28] An exploded side view of the unit according to the third embodiment.

[FIG. 29] A top view of a device according to a fourth embodiment.

[FIG. 30] A top view of a device according to a fifth embodiment.

[FIG. 31] A diagram illustrating an experimental example using the device according to the fifth embodiment.

[FIG. 32] A top view of an accommodation layer according to a first modified example.

[FIG. 33] A top view of an accommodation layer according to a second modified example.

[FIG. 34] A top view of an accommodation layer according to a third modified example.

[FIG. 35] A diagram illustrating a shape of through-holes in a flow passage forming member according to a fourth modified example.

[FIG. 36] A diagram illustrating a shape of through-holes in a flow passage forming member according to a fifth modified example.

[FIG. 37] A diagram illustrating a shape of through-holes in a flow passage forming member according to a sixth modified example.

[FIG. 38] A diagram illustrating a shape of through-holes in a flow passage forming member according to a seventh modified example.

[FIG. 39] A top view of a device according to a sixth embodiment.

[FIG. 40] A bottom view of the device according to the sixth embodiment.

[FIG. 41] A perspective view of a body and a base according to the sixth embodiment including a cross-section thereof.

[FIG. 42] A sectional view of the body and the base according to the sixth embodiment.

[FIG. 43] A diagram illustrating a flow of a liquid in the device according to the sixth embodiment.

[FIG. 44] A top view of the body according to the sixth embodiment.

[FIG. 45] A bottom view of the body according to the sixth embodiment.

[FIG. 46] A top view of the base according to the sixth embodiment.

[FIG. 47] A bottom view of the base according to the sixth embodiment.

[FIG. 48] A bottom view of a flow passage forming member according to the sixth embodiment.

[FIG. 49] A bottom view of an adhesive layer constituting the flow passage forming member according to the sixth embodiment.

[FIG. 50] A bottom view of a sheet constituting the flow passage forming member according to the sixth embodiment.

[FIG. 51] A diagram illustrating an example (a support layer) of a reticle pattern of an accommodation layer according to the sixth embodiment.

[FIG. 52] A diagram illustrating an example (a well layer) of the reticle pattern of the accommodation layer according to the sixth embodiment.

[FIG. 53] A diagram illustrating an example of an arrangement of sample chambers and liquid-passing ports in a microplate.

[FIG. 54] A diagram illustrating an example of the number of sample chambers when a ratio A:B of the number sample chambers A arranged in a Y direction to the number of sample chambers B arranged in an X direction in FIG. 53 is 2:3.

[FIG. 55] A diagram illustrating an example of the number of sample chambers when a ratio A:B of the number sample chambers A arranged in the Y direction to the number of sample chambers B arranged in the X direction in FIG. 53 is 2:1.5.

DESCRIPTION OF EMBODIMENTS

Hereinafter, embodiments of the present invention will be described with reference to the accompanying drawings. In the drawings, an XYZ coordinate system is illustrated according to necessity. In this specification, directions are defined on the basis of the XYZ coordinate system according to necessity. In embodiments, an X direction is an example of a first direction which is parallel to a horizontal plane. A Y direction is an example of a second direction which is perpendicular to the first direction in the horizontal plane. A Z direction is an example of a third direction which is perpendicular to the first direction and the second direction and which is parallel to a vertical direction. The +Z direction corresponds to an upper side in the vertical direction. The -Z direction corresponds to a lower side in the vertical direction. The drawings used for the following description may enlarge featured parts for the purpose of easy understanding of features of the present invention. In the drawings used for the following description, dimensions, ratios, and the like of constituents may not be the same as actual ones.

<First embodiment>

<Device>

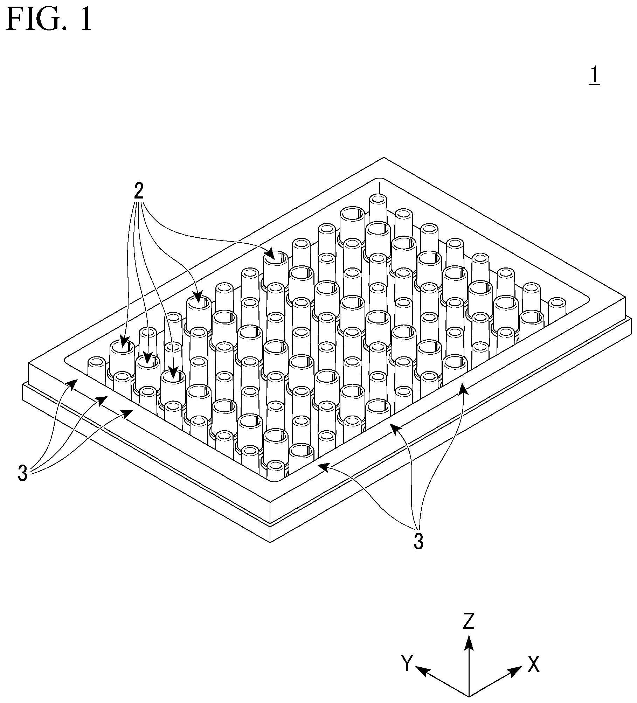

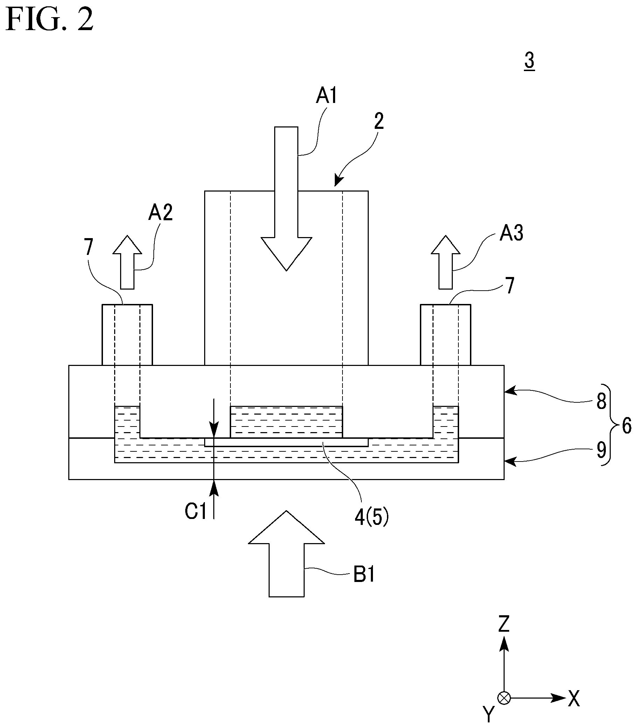

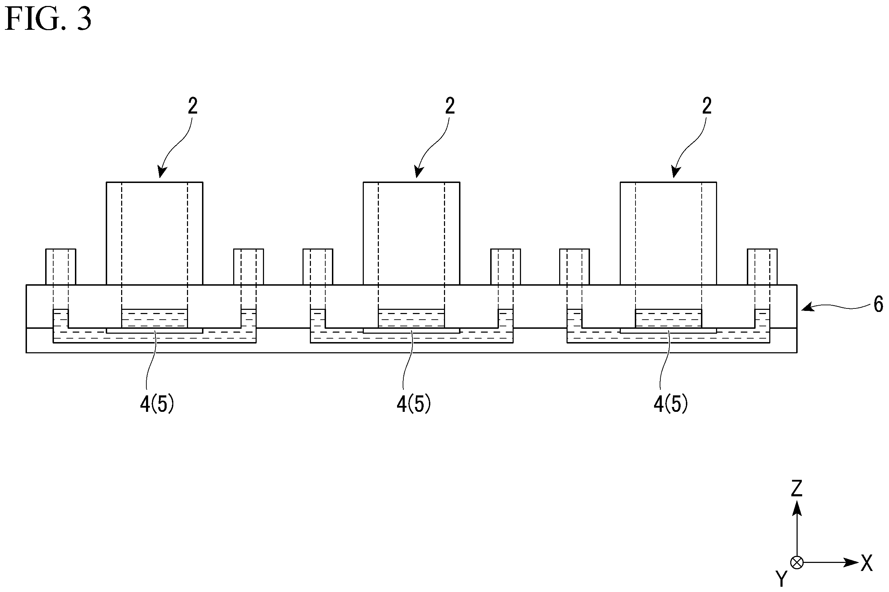

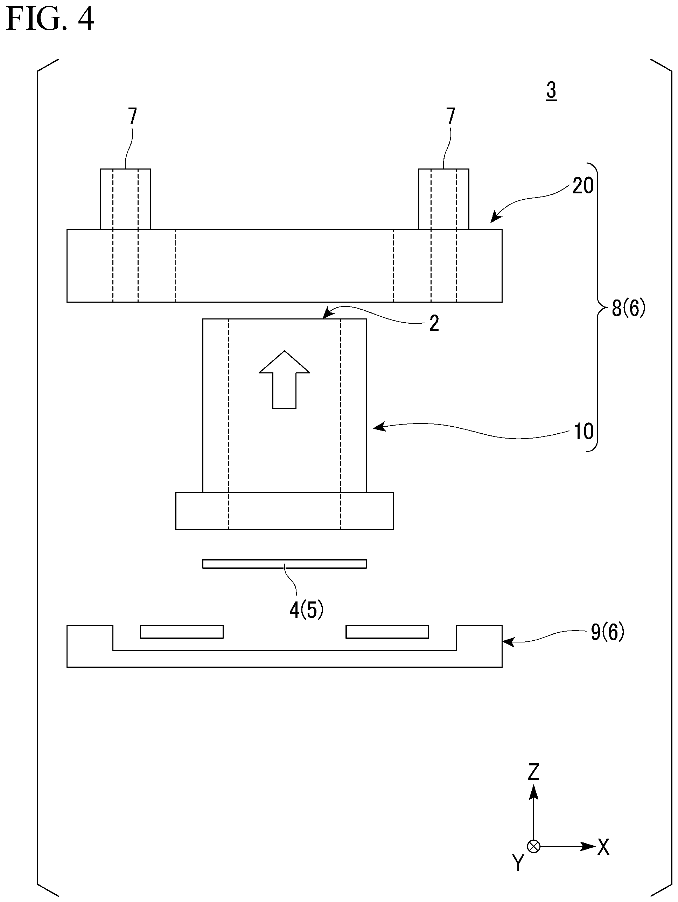

FIG. 1 is a perspective view of a device 1 according to a first embodiment. FIG. 2 is a diagram illustrating an example of high-magnification observation of a unit 3 according to the first embodiment. FIG. 3 is a diagram illustrating an example of a plurality of independent sample chambers 2 according to the first embodiment. FIG. 4 is an exploded view of a unit 3 according to the first embodiment.

As illustrated in FIGS. 1 to 4, the device 1 includes a plurality of units 3 of which each includes a sample chamber 2 that can individually accommodate a sample (cells or particles).

Each unit 3 includes an accommodation layer 5 including an accommodation portion 4 corresponding to the sample chamber 2 and a structure 6 for allowing a liquid to flow to the accommodation portion 4. The plurality of units 3 are independent such that there is no flow of a liquid therebetween. In FIG. 2, arrows A1, A2, and A3 indicate a flow direction of a liquid in the device 1, and an arrow B1 indicates an observation direction of samples (cells or particles) in the device 1.

<Structure>

The structure 6 includes a liquid-passing member 8 that includes a liquid-passing port 7 for allowing a liquid to flow to the accommodation portion 4 and to which the accommodation layer 5 is attached and a flow passage forming member 9 which is attached to the liquid-passing member 8 with the accommodation layer 5 interposed therebetween and which allows a liquid to flow between the sample chamber 2, the accommodation layer 5, and the liquid-passing port 7.

The structure 6 may include a liquid-passing member 8 which includes a liquid-passing port 7 for allowing a liquid to flow to the accommodation portion 4 and to which the accommodation layer 5 is attached. The structure 6 may include a flow passage forming member 9 which is attached to the liquid-passing member 8 with the accommodation layer 5 interposed therebetween and which allows a liquid to flow between the sample chamber 2, the accommodation layer 5, and the liquid-passing port 7.

For example, since a distance from the bottom surface of the device 1 to the accommodation layer 5 can be decreased as a thickness C1 of the flow passage forming member 9 is decreased, it is possible to facilitate high-magnification observation. In the present embodiment, since the bottom of the structure 6 is not a constituent taking charge of mechanical strength of the device 1, the thickness C1 of the flow passage forming member 9 can be decreased. It is possible to better facilitate decreasing the thickness C1 of the flow passage forming member 9 in comparison with a case in which the sample chamber and the flow passage are formed through mechanical fitting.

The liquid-passing member 8 includes a first member 10 including a sample chamber 2 and attached to the accommodation layer 5 and a second member 20 including a liquid-passing port 7 and assembled into the first member 10. In the present embodiment, the first member 10 and the second member 20 are separate components. The first member 10 and the second member 20 are detachably attached to each other.

The liquid-passing member 8 may include a first member 10 including a sample chamber 2 and attached to the accommodation layer 5. The liquid-passing member 8 may include a second member 20 including a liquid-passing port 7 and assembled into the first member 10.

The device 1 according to the present embodiment has the same rectangular outer shape as a microplate in a plan view. The microplate is a plate-like member including a plurality of cavities (holes or wells). The microplate is an experimental and test instrument, and each well is used as a test tube or a petri dish. For example, as a size of the microplate in a plan view, a length in a long-side direction (the X direction) (an outer dimension of the long side) is 127.76 mm (about 128 mm), and a length of in a short-side direction (the Y direction) (an outer dimension of the short side) is 85.48 mm (about 85 mm).

A sample chambers 2 are provided in the short-side direction (the Y direction) of the device 1, and B sample chambers 2 are provided in the long-side direction (the X direction) of the device 1. In the present embodiment, a ratio A:B of the number of sample chambers 2 A arranged in the short-side direction in the device 1 to the number of sample chambers 2 B arranged in the long-side direction is 2:1. The ratio A:B is not limited thereto and can be changed according to design specifications.

The device 1 may have the same rectangular outer shape as the microplate in a plan view. A sample chambers 2 may be provided in the short-side direction (the Y direction) of the device 1. B sample chambers 2 may be provided in the long-side direction (the X direction) of the device 1. The ratio A:B of the number of sample chambers 2 A arranged in the short-side direction in the device 1 to the number of sample chambers 2 B arranged in the long-side direction may be 2:3, 2:1.5, or 2:1.

In the present embodiment, the number of sample chambers 2 A arranged in the short-side direction in the device 1 is 8, and the number of sample chambers 2 B arranged in the long-side direction is 4. 32 sample chambers 2 are provided in the device 1. The number of sample chambers 2 provided in the device 1 is not limited thereto and can be changed according to design specifications.

<First member>

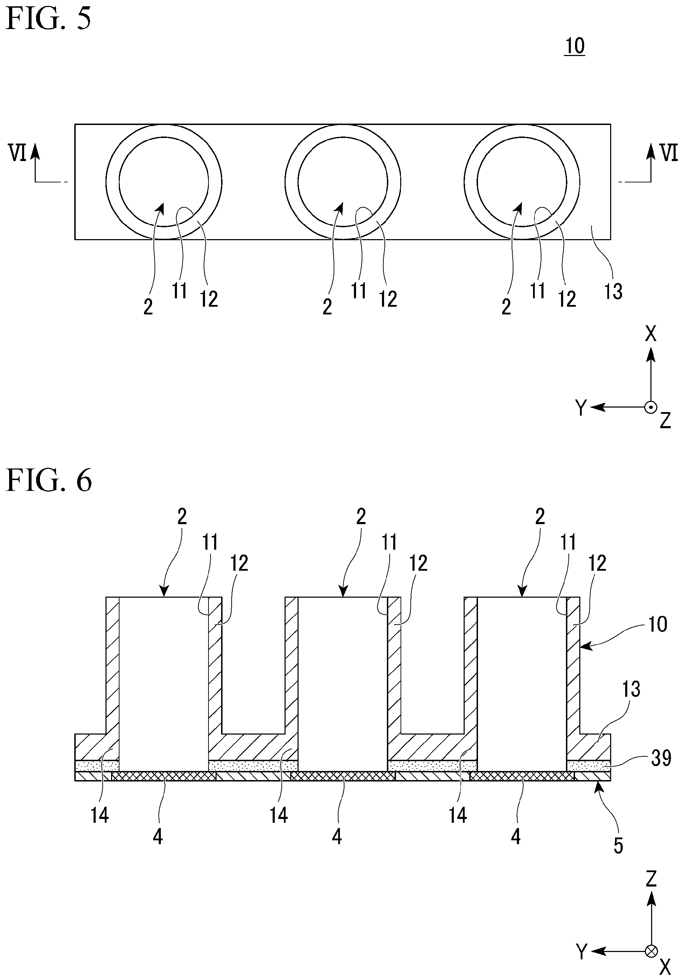

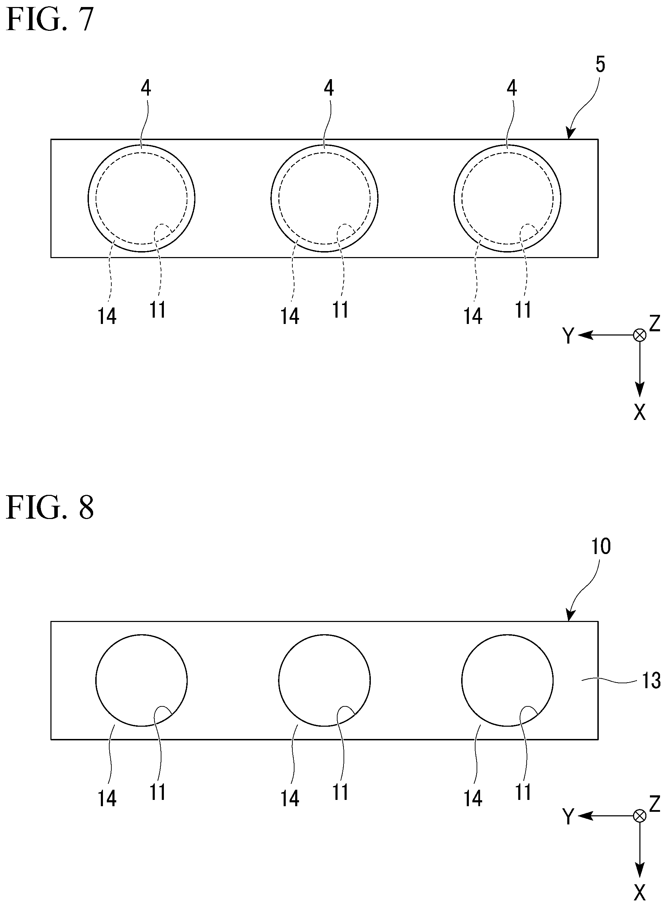

FIG. 5 is a top view of the first member 10 according to the first embodiment. FIG. 6 is a diagram illustrating an accommodation layer 5 according to the first embodiment along with a cross-section taken along line VI-VI in FIG. 5. FIG. 7 is a bottom view of the accommodation layer 5 according to the first embodiment. FIG. 8 is a bottom view of the first member 10 according to the first embodiment.

In the illustrated example, the first member 10 including three sample chambers 2 is illustrated for the purpose of convenience of illustration. In reality, the first member 10 includes six or more sample chambers 2. The number of sample chambers 2 included in the first member 10 is not limited thereto and can be changed according to design specifications.

A material of the first member 10 can be arbitrarily selected according to a subject accommodated in the sample chambers 2. When cells are accommodated in the sample chambers 2, it is preferable that the material of the first member 10 do not have cytotoxicity (for example, it needs to be ascertained that there is no cytotoxicity in a cytotoxicity test in ISO10993-5 and a standard similar thereto), and a cytotoxic component not be eluted in an aqueous solution or alcohol. The material of the first member 10 is preferably a material which has low autofluorescence, which is not significantly degraded through sterilization treatment such as gamma irradiation, ethylene oxide steam exposure, or ultraviolet irradiation, and which does not dissolve in alcohol.

The first member 10 can additionally include one or more materials selected from a group consisting of a material not having cytotoxicity, a material in which a cytotoxic component is not eluted, a material having low autofluorescence, a material which is not significantly degraded by gamma irradiation, a material which is not significantly degraded by ethylene oxide steam exposure, a material which is not significantly degraded by ultraviolet irradiation, a material which is not significantly degraded by sterilization treatment, a material which does not dissolve in alcohol, and/or mixtures thereof and preferably includes a material which does not have cytotoxicity and in which a cytotoxic component is not eluted.

The color of the first member 10 can be arbitrarily selected according to a subject accommodated in the sample chambers 2. The color of the first member 10 is preferably black in view of reduction of autofluorescence of the material and an influence of reflection of light at the time of microscope observation.

Referring to FIGS. 5 to 8 together, the first member 10 includes a tube-like tubular wall portion 12 including a sample chamber opening 11 of a size through which a sample (a cell or a particle) is able to pass (for example, through which a liquid sample can be added with a pipette) and a plate-like tabular wall portion 13 to which the tubular wall portion 12 is connected. The first member 10 has a structure in which three tubular wall portions 12 are connected on the tabular wall portion 13. The number of tubular wall portions 12 (sample chambers 2) provided on the tabular wall portion 13 is not limited thereto and can be changed according to design specifications.

The tubular wall portion 12 has a cylindrical shape. The shape of each sample chamber 2 is a cylinder. The shape of the sample chamber 2 is not limited thereto and can be changed according to design specifications. For example, the shape of the sample chamber 2 may be an elliptic cylinder, an oval cylinder, or a polygonal pillar. For example, the shape of the sample chamber 2 is preferably a prefect-circle cylinder. For example, an opening diameter of the upper part of the sample chamber 2 and an opening diameter of a lower part of the sample chamber 2 (a bottom part) may be the same or different from each other. For example, the shape of the sample chamber 2 may be a conical shape in which the opening diameter of the lower part (bottom part) of the sample chamber 2 is smaller than the opening diameter of the upper part of the sample chamber 2.

For example, the shape of an opening of the sample chamber 2 may be a conical shape or a pyramidal shape. For example, the shape of the opening of the sample chamber 2 is not limited thereto and can be changed according to design specifications.

The thickness of the tubular wall portion 12 is not limited as long as mechanical strength of the tubular wall portion 12 can be maintained. The thickness of the tubular wall portion 12 corresponds to a gap between an inner circumference and an outer circumference (a difference between an inner radius and an outer radius) of the tubular wall portion 12. For example, the thickness of the tubular wall portion 12 is preferably equal to or greater than 0.1 mm and equal to or less than 25 mm.

The inner diameter (inner dimension) of the sample chamber opening 11 is not limited as long as a sample (a cell or a particle) is able to pass therethrough. For example, the inner diameter of the sample chamber opening 11 is equal to or greater than 1 mm and equal to or less than 50 mm. For example, the inner diameter of the sample chamber opening 11 is preferably equal to or less than 30 mm in view of curbing distortion of the accommodation layer 5. For example, in consideration of easiness of observation, the inner diameter of the sample chamber opening 11 is more preferably equal to or greater than 1.5 mm and equal to or less than 10 mm.

The height of the sample chamber 2 is not limited as long as a volume for accommodating a liquid can be secured. The height of the sample chamber 2 corresponds to a length between an upper end and a lower end (a length in the Z direction) of the tubular wall portion 12. In other words, the height of the sample chamber 2 corresponds to a distance between the top surface of the tabular wall portion 13 and the upper end of the first member 10. For example, in consideration of operability, the height of the sample chamber 2 is preferably equal to or greater than 0 mm and equal to or less than 30 mm. Since the tabular wall portion 13 has a thickness, the height of the sample chamber 2 may be 0 mm.

A hole portion 14 to which the accommodation layer 5 is attached is formed in the tabular wall portion 13. The shape of the hole portion 14 is a cylindrical shape corresponding to the shape of the sample chamber 2. The shape in a plan view of the outer shape of the tabular wall portion 13 is rectangular. For example, as a size in a plan view of the tabular wall portion 13, a length in the long-side direction (an outer dimension of the long side) is equal to or greater than 4.5 mm, and a length in the short-side direction (an outer dimension of the short side) is equal to or greater than 1.5 mm. For example, the length in the long-side direction of the tabular wall portion 13 is not limited as long as it is a length equal to or less than the length in the long-side direction (127.76 mm) of the microplate. For example, the length in the short-side direction of the tabular wall portion 13 is not limited as long as it is equal to or greater than a length obtained by adding the dimension of the sample chamber opening 11 to an attachment dimension of the accommodation layer 5 (a dimension remaining for pasting to attach the accommodation layer 5 thereto).

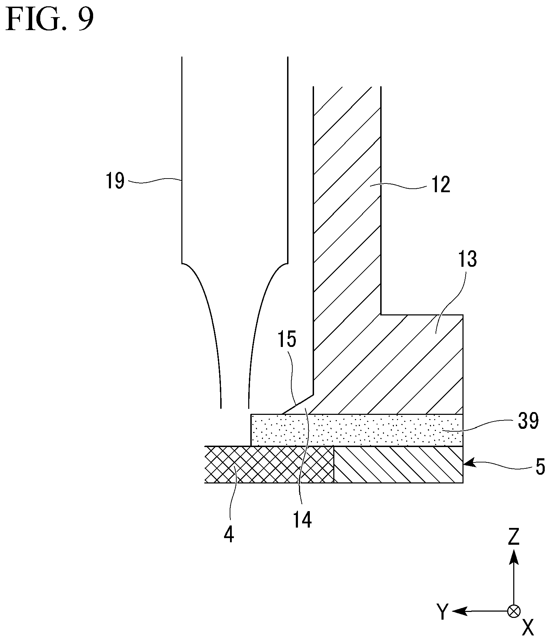

FIG. 9 is a diagram illustrating a capillary 19 for recovering contents of the accommodation portion 4 along with a partially enlarged view of FIG. 6.

Referring to FIG. 9 together, for example, an edge 15 (a lower inner circumferential edge) of the hole portion 14 in the tabular wall portion 13 preferably has an inclination. In the illustrated example, the edge 15 of the hole portion 14 is inclined such that the edge is located more inward in da radial direction as it goes to the -Z side. By adding an inclination to the edge 15 of the hole portion 14 in the tabular wall portion 13, a pick-up capillary 19 can access the corner (outermost circumference) of the accommodation portion 4 at the time of picking-up of a sample (a cell or a particle).

<Accommodation layer>

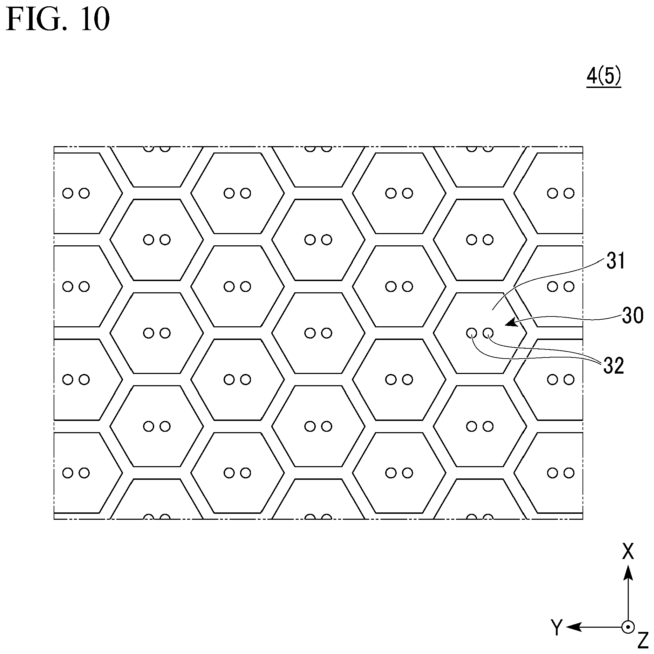

FIG. 10 is a diagram illustrating the accommodation layer 5 (an example of a well array filter) according to the first embodiment.

Referring to FIG. 10 together, the accommodation layer 5 is a film-like member including the accommodation portion 4 corresponding to the sample chamber 2. The accommodation layer 5 according to the present embodiment is a well array filter including a well opening 30 of a size through which a sample (a cell or a particle) is able to pass and includes a well bottom 31 corresponding to the well opening 30. The well bottom 31 includes a through-hole 32 allowing only a liquid to pass therethrough.

The accommodation layer 5 may include a well opening 30 of a size through which a sample (a cell or a particle) is able to pass. The accommodation layer 5 may include a well bottom 31 corresponding to the well opening 30. The well bottom 31 may include a through-hole 32 allowing only a liquid to pass therethrough.

In the illustrated example, the shape in a plan view of the well opening 30 is hexagonal and may be any shape in a plan view as long as it has a size through which a sample (a cell or a particle) is able to pass. In the illustrated example, two through-holes 32 which are circular in a plan view are provided at the center of the well bottom 31, and the shape in a plan view of the through-hole 32 and the number of through-holes 32 are not limited as long as it can allow only a liquid to pass therethrough.

The accommodation layer 5 is not limited as long as it can accommodate a sample (a cell or a particle). For example, the accommodation layer 5 may include a through-hole 32 allowing only a liquid to pass therethrough in the bottom of a well with an opening diameter of a cell size. For example, the accommodation layer 5 may not include a through-hole 32 in the bottom of the well. For example, the accommodation layer 5 may include a through-hole 32 of a size through which a cell is not allowed to pass in the bottom of the well. For example, when a cell is accommodated as a single cell, a well structure of a cell size including a through-hole 32 in the bottom of the well is most preferably employed.

The accommodation layer 5 according to the present embodiment is formed of a negative resist. The material of the accommodation layer 5 can be arbitrarily selected according to a subject accommodated in the sample chambers 2. When the well structure is employed or through-holes 32 of a size through which a cell is not able to pass are regularly arranged, the material of the accommodation layer 5 is preferably a negative photoresist or polyethylene glycol or polydimethyl siloxane (PDMS) having a polymerizable functional group and is more preferably a negative photoresist.

The accommodation layer 5 may additionally include one or more materials selected from a group consisting of a negative photoresist, polyethylene glycol or polydimethyl siloxane (PDMS) having a polymerizable functional group, and/or mixtures thereof and preferably includes a negative photoresist.

The surface of the accommodation layer 5 may be coated with a non-cell-adhesion polymer or a cell-adhesion factor. Examples of the non-cell-adhesion polymer include 2-methacryloxy ethyl phosphorylcholine (MPC) polymer, polyoligoehtylene glycol methacrylate (PEG), poly-2-methoxy ethyl acrylate (PMEA), and polyvinyl alcohol (PVA).

For example, when a plurality of sample chambers 2 are provided in the first member 10, the accommodation portion 4 is preferably provided in each sample chamber 2 by attaching one film including the accommodation portions 4 having a shape along the first member 10 to the bottom of the first member 10. In the present embodiment, one film including the accommodation portions 4 at only positions corresponding to the sample chambers 2 is attached to the bottom surface of the first member 10.

For example, when the accommodation layer 5 has a porous structure such as a well structure and an adhesive is used to cause the accommodation layer 5 to adhere to the first member 10, the adhesive enters the inside of wells, and thus an extra adhesive is needed. When an adhesive sheet is used, there is a high likelihood that a sufficient contact area between the adhesive sheet and the accommodation layer 5 is not secured and thus sufficient adhesion strength cannot be obtained. In order to avoid this, it is preferable that the accommodation portion 4 be provided in only a part of the accommodation layer 5 corresponding to the sample chamber 2 and the other part be a flat portion which is flat. By employing this structure, it is possible to obtain sufficient adhesion strength with a small amount of adhesive and to secure a sufficient contact area between an adhesive sheet and the accommodation layer 5 even when the adhesive sheet is used.

For example, when the accommodation portion 4 is provided in only a part of the accommodation layer 5 corresponding to the sample chamber 2, a size of the accommodation portion 4 in a plan view can be set to be larger than the opening of the lower part (the bottom part) of the sample chamber 2. Accordingly, it is possible to provide the accommodation layer 5 in the whole sample chamber 2.

For example, when the accommodation portion 4 is provided in only a part of the accommodation layer 5 corresponding to the sample chamber 2, an adhesive layer 39 can be provided on the outer circumference of the accommodation portion 4. Accordingly, since the accommodation portion 4 and the first member 10 can be caused to adhere to each other satisfactorily, it is possible to curb liquid leakage. For example, when the adhesive layer 39 is provided along the whole outer circumference of the accommodation portion 4, it is possible to more effectively curb liquid leakage.

<Adhesive layer>

The adhesive layer 39 is provided on the bottom surface of the first member 10 to cause a film (the accommodation layer 5 including the accommodation portion 4) to adhere thereto. The adhesive layer 39 may be provided on the whole bottom surface of the first member 10 or may be provided in only the periphery of the bottom of the sample chamber opening 11.

The adhesive layer 39 may be provided by applying a liquid adhesive such as an epoxy system or an acryl system using an arbitrary method, or an adhesive sheet including an adhesive layer 39 on both sides thereof may be used. For example, when the surfaces of the first member 10 and the film (the accommodation layer 5 including the accommodation portion 4) can adhere to each other through direct chemical and physical reforming, both may be caused to adhere using this method.

The material of the adhesive layer 39 can be arbitrarily selected according to a subject accommodated in the sample chambers 2. When cells are accommodated in the sample chamber 2, it is preferable that the material of the adhesive layer 39 do not have cytotoxicity (for example, it needs to be ascertained that there is no cytotoxicity in a cytotoxicity test in ISO10993-5 and a standard similar thereto), and a cytotoxic component be not eluted in an aqueous solution or alcohol. The material of the adhesive layer 39 is preferably a material which has low autofluorescence, which does not remarkably degenerate through sterilization treatment such as gamma irradiation, ethylene oxide steam exposure, or ultraviolet irradiation, and which does not dissolve in alcohol. A reason for resistance to alcohol is that ethanol is used for surface treatment of the accommodation layer 5 (for example, surface treatment of the accommodation layer 5 itself or the first member 10 having the accommodation layer 5 attached thereto) at the time of manufacturing and pre-wetting treatment of the accommodation layer 5 at the time of use.

The adhesive layer 39 can additionally include one or more materials selected from a group consisting of a material not having cytotoxicity, a material in which cytotoxic components are not eluted, a material having low autofluorescence, a material which is not significantly degraded by gamma irradiation, a material which is not significantly degraded by ethylene oxide steam exposure, a material which is not significantly degraded by ultraviolet irradiation, a material which is not significantly degraded by sterilization treatment, a material which does not dissolve in alcohol, and/or mixtures thereof and preferably includes a material which does not have cytotoxicity and in which cytotoxic components are not eluted.

For example, when the film (the accommodation layer 5 including the accommodation portion 4) is attached to the first member 10, the adhesive layer 39 is caused to protrude into the sample chamber 2. Accordingly, since a gap between the first member 10 and the film (the accommodation layer 5 including the accommodation portion 4) can be removed, a sample (a cell or a particle) does not enter the gap.

<Second member>

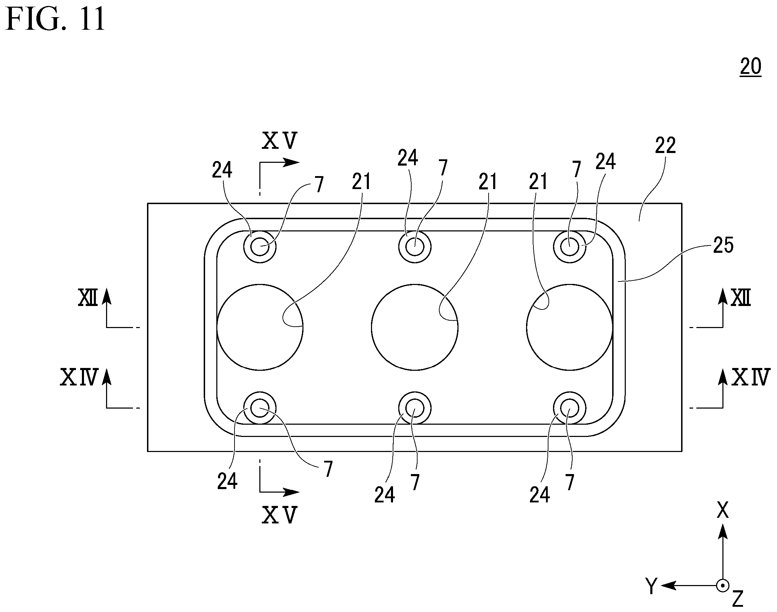

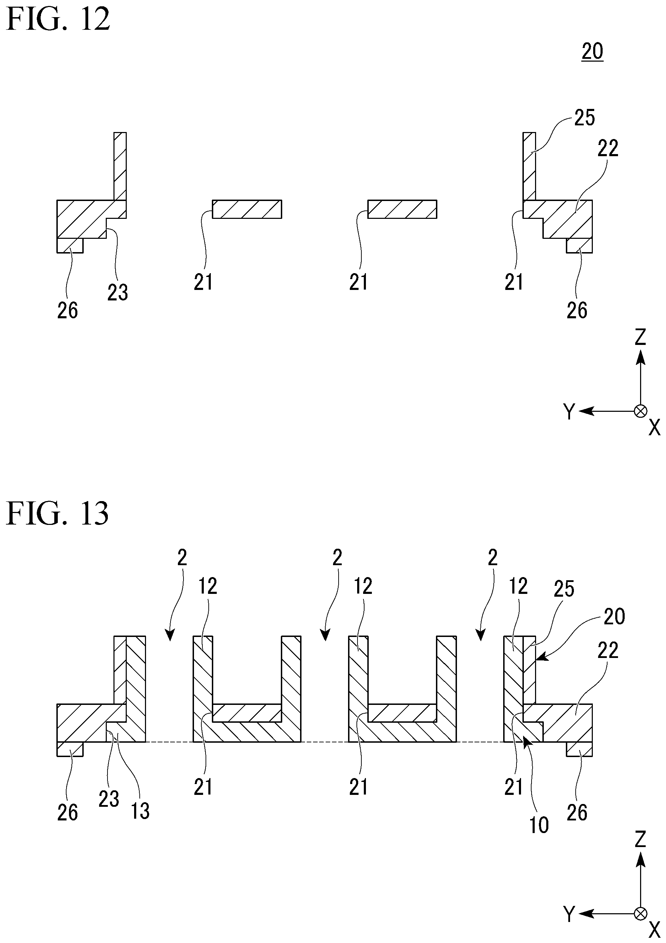

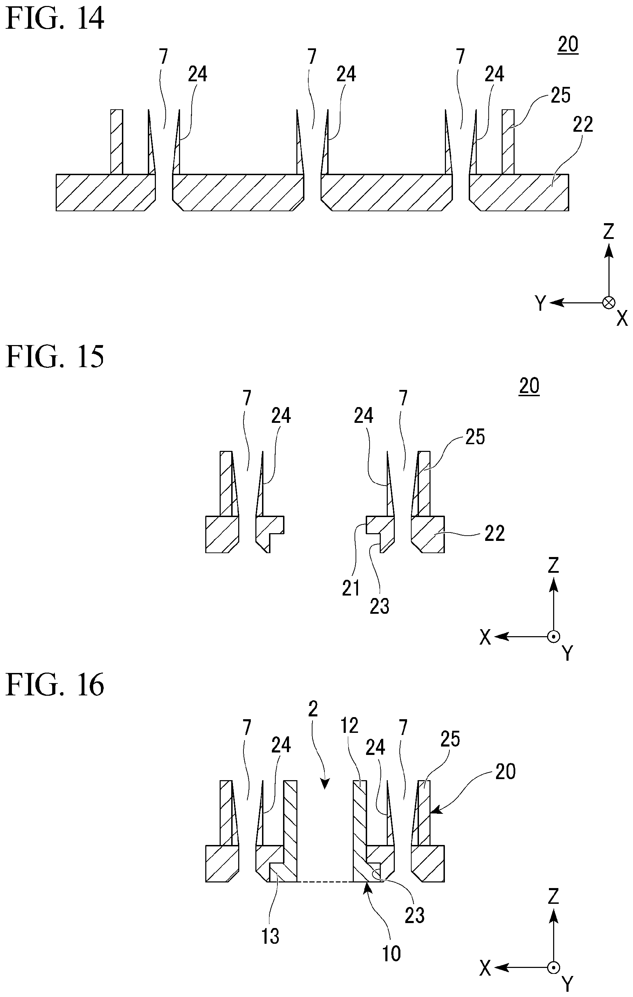

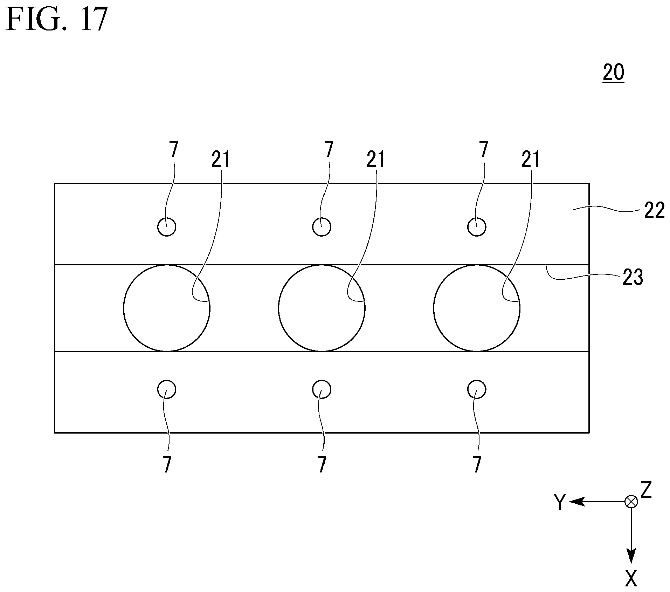

FIG. 11 is a top view of the second member 20 according to the first embodiment. FIG. 12 is a sectional view taken along line XII-XII in FIG. 11. FIG. 13 is a diagram illustrating the first member 10 along with the sectional view of FIG. 12. FIG. 14 is a sectional view taken along line XIV-XIV in FIG. 11. FIG. 15 is a sectional view taken along line XV-XV in FIG. 11. FIG. 16 is a diagram illustrating the first member 10 along with the sectional view of FIG. 15. FIG. 17 s a bottom view of the second member 20 according to the first embodiment.

Referring to FIGS. 11 to 17 together, the second member 20 constitutes a main body of the device 1. The second member 20 is a member which includes the liquid-passing port 7 and which is assembled into the first member 10.

The second member 20 includes a fixing wall portion 22 including a hole 21 into which the tubular wall portion 12 of the first member 10 is inserted and fixed. The fixing wall portion 22 includes a groove 23 into which the tabular wall portion 13 of the first member 10 is fitted and fixed in the bottom surface thereof. The hole 21 of the fixing wall portion 22 is formed at a position corresponding to the sample chamber 2 of the first member 10. An outer shape of the groove 23 of the fixing wall portion 22 is rectangular in a plan view such that the tabular wall portion 13 of the first member 10 is fitted thereto.

For example, when the first member 10 and the second member 20 are assembled, the first member 10 is inserted into the hole 21 of the fixing wall portion 22 of the second member 20 from the bottom side of the second member 20 (for example, the arrow direction in FIG. 4). Accordingly, the tubular wall portion 12 of the first member 10 is inserted into the hole 21 of the fixing wall portion 22 of the second member 20, and the tabular wall portion 13 of the first member 10 is fitted into the groove 23 of the fixing wall portion 22. For example, the bottom surfaces of the first member 10 and the second member 20 which are assembled are flush with each other.

One or more liquid-passing ports 7 can be provided around the hole 21 of the fixing wall portion 22 (a hole corresponding to the sample chamber 2). The second member 20 includes a tube-like liquid-passing tubular portion 24 including the liquid-passing port 7. A through-hole which is open for the tip of the liquid-passing tubular portion 24 to pass therethrough may be formed in the second member 20. In the illustrated example (the top view of FIG. 11), a pair of liquid-passing ports 7 is formed at positions which are symmetric with respect to the hole 21 of the fixing wall portion 22 (the hole corresponding to the sample chamber 2) in a plan view.

The liquid-passing port 7 is not limited as long as it is a hole allowing a liquid to flow to the accommodation portion 4, and the number of liquid-passing ports 7 which are installed is not limited. For example, two liquid-passing ports 7 are provided at positions which are symmetric with respect to the hole 21 of the fixing wall portion 22 (the hole corresponding to the sample chamber 2). Accordingly, since a liquid can be picked up simultaneously form both sides of the sample chamber 2, samples (cells or particles) can be accommodated in the accommodation layer 5 without being biased. By putting a liquid into one of the two liquid-passing ports 7 and picking up a liquid from the other liquid-passing port 7 using a pump or the like, it is possible to perfuse the sample chamber 2 and the flow passage with a liquid.

For example, when two liquid-passing ports 7 are provided symmetric with respect to the sample chamber 2 in a plan view, a center distance between the liquid-passing ports 7 can be set to an integer multiple of a center distance between two neighboring channels in a multi-channel pipette. Accordingly, it is possible to simultaneously pick up a liquid from two liquid-passing ports 7 using two channels of the multi-channel pipette.

In the illustrated example (the sectional view of FIG. 14), an upper opening of the liquid-passing port 7 is formed in a hole of a conical shape corresponding to a tip shape of a pipette tip with a volume of 100 μL. The bottom of the liquid-passing port 7 (the inner diameter of the liquid-passing tubular portion 24 of the second member 20) has a diameter corresponding to an opening diameter of the tip of the pipette tip.

The second member 20 includes a surrounding wall portion 25 of a rectangular frame including rounded corners in a plan view on the top surface of the fixing wall portion 22. The surrounding wall portion 25 is provided to surround the sample chamber 2 and the liquid-passing port 7 as a whole in a plan view. For example, a lid member (not illustrated) covering the sample chamber 2 and the liquid-passing port 7 as a whole is detachably attached to the surrounding wall portion 25 in order to prevent vaporization of a sample. For example, the surrounding wall portion 25 also serves as a grip for fixing the device 1 at the time of loading the sample.

For example, a rib 26 for preventing the bottom surface of the device 1 from coming into contact with the ground may be provided on the bottom of the second member 20. In the illustrated example (the sectional view of FIG. 12), the rib 26 is provided at an end of the short side (an outer end in the Y direction) of the bottom of the second member 20. For example, a height (a length in the Z direction) of the rib 26 is preferably equal to or less than 1 mm in order to enable microscope observation with a high magnification.

For example, a lower shape (corresponding to the through-hole of the bottom surface of the fixing wall portion 22) of the liquid-passing port 7 is a circular shape or a polygonal pyramidal shape. For example, the lower shape of the through-hole of the liquid-passing port 7 is preferably a conical shape or a polygonal pyramidal shape. Accordingly, bubbles are less likely to gather in the flow passage.

\<Flow passage forming member\>

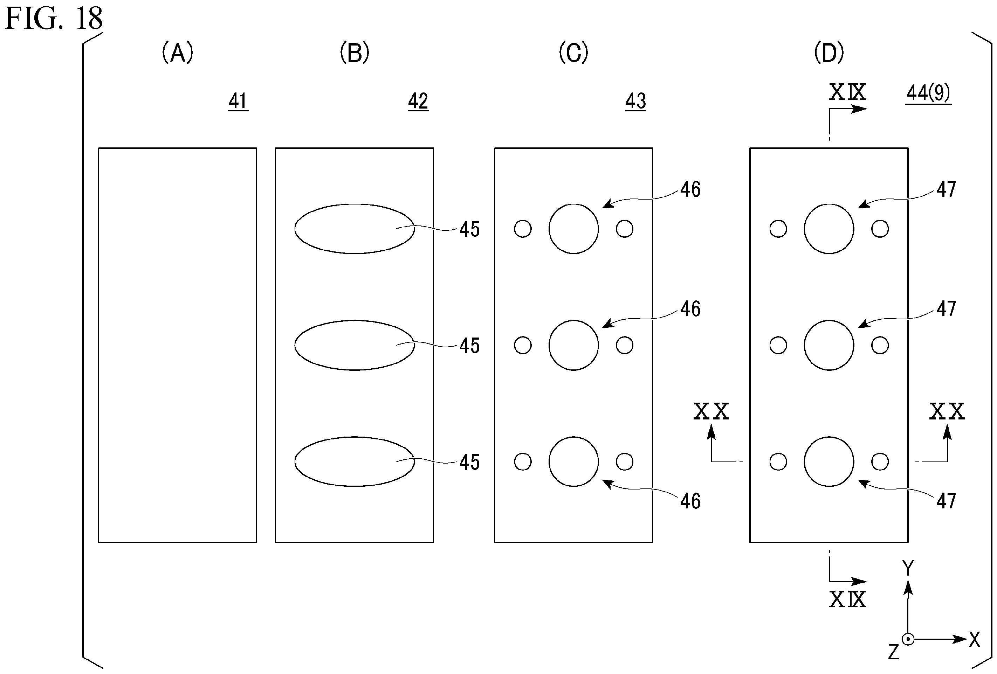

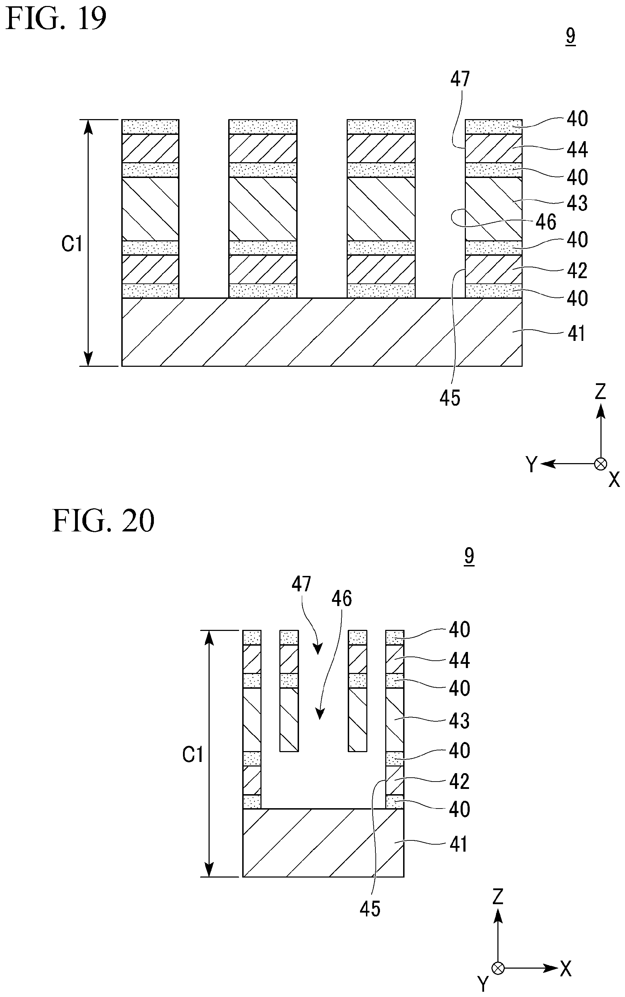

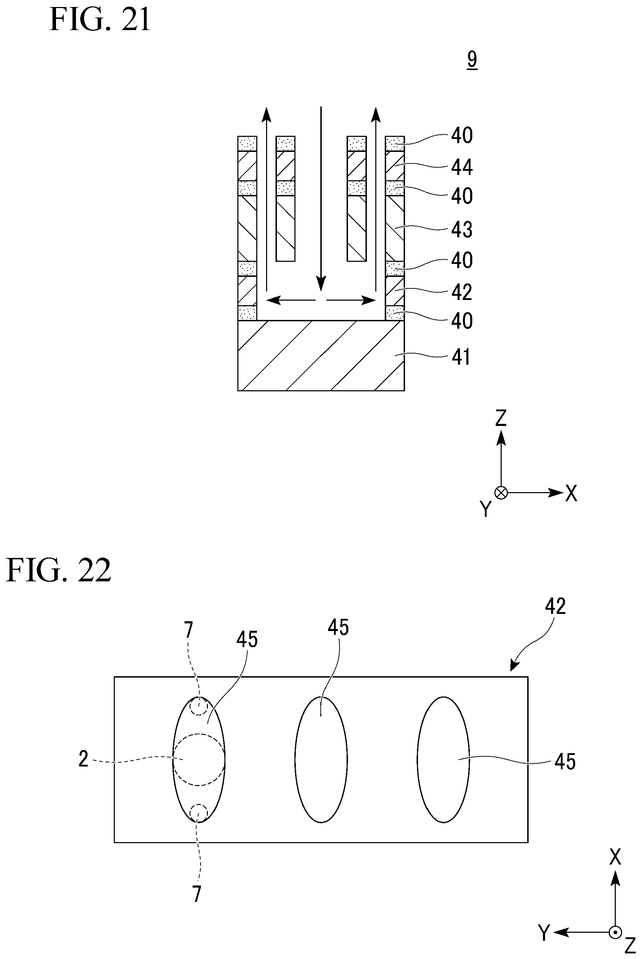

FIG. 18 is a diagram illustrating the configuration of the flow passage forming member 9 according to the first embodiment. FIG. 18(A) is a top view of a first sheet 41, FIG. 18(B) is a top view of a second sheet 42, FIG. 18(C) is a top view of a third sheet 43, and FIG. 18(D) is a top view of a fourth sheet 44. FIG. 19 is 1 sectional view of the flow passage forming member 9 in the cross-section taken along XIX-XIX in FIG. 18. FIG. 20 is a sectional view of the flow passage forming member 9 in the cross-section taken along XX-XX in FIG. 18. FIG. 21 is a diagram illustrating an example of a flow of a liquid in the flow passage forming member 9 according to the first embodiment. FIG. 22 is a diagram illustrating a shape of communication holes 45 in the flow passage forming member 9 according to the first embodiment.

Referring to FIGS. 18 to 22 together, the flow passage forming member 9 is attached to the liquid-passing member 8 with the accommodation layer 5 interposed therebetween. The flow passage forming member 9 is a structure for allowing a liquid to flow between the sample chamber 2, the accommodation layer 5, and the liquid-passing port 7. When a plurality of sample chambers 2 are provided, the flow passage forming member 9 is for preventing a liquid from flowing between the sample chambers 2 and making the sample chambers 2 independent.

The flow passage forming member 9 according to the present embodiment includes a first sheet 41 which is transparent, a second sheet 42 which is stacked on the first sheet 41 and includes a communication hole 45 connecting the liquid-passing port 7 and the sample chamber 2, a third sheet 43 which is stacked on the second sheet 42 and includes an opening hole 46 at positions of the liquid-passing member 8 corresponding to the liquid-passing port 7 and the sample chamber 2, and a fourth sheet 44 which is stacked on the third sheet 43 and has the same shape in a plan view as the third sheet 43. The fourth sheet 44 includes an opening hole 47 at positions corresponding to the opening holes 46 of the third sheet 43. An adhesive layer 40 is provided on both sides of the second sheet 42 and both sides of the fourth sheet 44.

The flow passage forming member 9 may include a first sheet 41 which is transparent. The flow passage forming member 9 may include a second sheet 42 which is stacked on the first sheet 41 and includes a communication hole 45 connecting the liquid-passing port 7 and the sample chamber 2. The flow passage forming member 9 may include a third sheet 43 which is stacked on the second sheet 42 and includes an opening hole 46 at positions of the liquid-passing member 8 corresponding to the liquid-passing port 7 and the sample chamber 2. The flow passage forming member 9 may include a fourth sheet 44 which is stacked on the third sheet 43 and has the same shape in a plan view as the third sheet 43. An adhesive layer 40 may be provided on both sides of the second sheet 42. An adhesive layer 40 may be provided on both sides of the fourth sheet 44.

For example, a thickness C1 of the flow passage forming member 9 is equal to or less than 2 mm. For example, in order to enable high-magnification observation using an inverted microscope (an example of a microscope), the thickness C1 of the flow passage forming member 9 is preferably equal to or less than 1 mm and more preferably equal to or less than 0.5 mm. The thickness C1 of the flow passage forming member 9 corresponds to a distance from the bottom surface of the device 1 to the accommodation layer 5 (see FIG. 2).

The thickness C1 of the flow passage forming member 9 is preferably equal to or less than 1.5 mm, more preferably equal to or less than 1.0 mm, and still more preferably equal to or less than 0.5 mm.

In order to decrease the thickness C1 of the flow passage forming member 9, it is preferable that the flow passage forming member 9 be manufactured by stacking a film. By stacking the first sheet 41 without any hole, the second sheet 42 including the communication hole 45 connecting the liquid-passing port 7 and the sample chamber 2, the third sheet 43 including the opening hole 46 at the positions of the liquid-passing member 8 corresponding to the liquid-passing port 7 and the sample chamber 2, and the fourth sheet 44 having the same shape in a plan view as the third sheet 43 in the Z direction, it is possible to manufacture the flow passage forming member 9. For example, by causing the top surface of the flow passage forming member 9 to adhere to the bottom surfaces of the first member 10 and the second member 20 which are assembled, a liquid can flow between the liquid-passing port 7 and the sample chamber 2.

The shape of the communication hole 45 of the second sheet 42 is not limited as long as it is a shape which includes a part with a larger width than the hole 21 of the fixing wall portion 22 (the hole corresponding to the sample chamber 2 in the second member 20) in a plan view and which can surround the openings of the sample chamber 2 and the liquid-passing port 7. In the present embodiment, the shape in a plan view of the communication hole 45 of the second sheet 42 is an elliptical shape surrounding the openings of the sample chamber 2 and the liquid-passing port 7. The shape of the communication hole 45 of the second sheet 42 is a shape in which the width on the liquid-passing port 7 side is smaller than the width on the sample chamber 2 side in a plan view. With this configuration, it is possible to minimize a volume of the flow passage. The shape in a plan view of the communication hole 45 of the second sheet 42 is not limited thereto and can be changed according to design specifications.

A volume of the communication hole 45 of the second sheet 42 is calculated on the basis of an area in a plan view of the communication hole 45 and a depth of the communication hole 45 (the thickness of the second sheet 42). The volume of the communication hole 45 of the second sheet 42 is set to a volume smaller than the volume of the sample chamber 2 of the first member 10.

When a plurality of sample chambers 2 are provided in one device 1, the third sheet 43 including an opening hole 46 at positions of the liquid-passing member 8 corresponding to the liquid-passing port 7 and the sample chamber 2 can be stacked in addition to the first sheet 41 and the second sheet 42. Accordingly, it is possible to prevent a liquid from flowing between the sample chambers 2 along the fitting part between the first sheet 41 and the second sheet 42.

The sheets constituting the flow passage forming member 9 may be provided as one film (a single-layered member) or may be provided as a stacked member of a plurality of films. For example, the configuration of the sheets (the number of films) constituting the flow passage forming member 9 can be changed according to design specifications.

The sheets and the films may adhere to and be stacked on each other by providing the adhesive layer 40 on at least one side of the sheets or the films. It is preferable that the material of the adhesive layer 40 do not have cytotoxicity (for example, it needs to be ascertained that there is no cytotoxicity in a cytotoxicity test in ISO10993-5 and a standard similar thereto), and cytotoxic components not be eluted in an aqueous solution or alcohol. For example, when the sheets and the films have thermos-plasticity, the stacked sheets or films may be adhered to each other and stacked using a heating press.

The adhesive layer 40 can additionally include one or more materials selected from a group consisting of a material not having cytotoxicity, a material in which cytotoxic components are not eluted, and/or mixtures thereof and preferably includes a material which does not have cytotoxicity and in which cytotoxic component are not eluted.

For example, the thicknesses of the first sheet 41 and the second sheet 42 are arbitrary as long as the total thickness of the flow passage forming member 9 is the thickness C1 (for example, equal to or less than 2 mm). For example, the thickness of the first sheet 41 is about 188 $\mu$m. For example, the thickness of the second sheet 42 is about 50 $\mu$m. For example, the thickness of the third sheet 43 is the same as the thickness of the first sheet 41 (about 188 $\mu$m). For example, the thickness of the fourth sheet 44 is the same as the thickness of the second sheet 42 (about 50 $\mu$m).

For example, the first sheet 41 is transparent in a wavelength range from blue to infrared which is used for excitation of a fluorescence microscope. For example, the first sheet 41 is formed of a material which does not emit fluorescence and which is not colored, clouded, or degenerated even through sterilization treatment such as gamma irradiation, ultraviolet irradiation, or ethylene oxide steam exposure. For example, the material of the first sheet 41 is preferably a cycloolefin copolymer (COC) or a cycloolefin polymer (COP).

The first sheet 41 can additionally include one or more materials selected from a group consisting of a material which does emit fluorescence, a material which is not colored, clouded, or degenerated even through gamma irradiation, a material which is not colored, clouded, or degenerated even through ultraviolet irradiation, a material which is not colored, clouded, or degenerated even through ethylene oxide stem exposure, a material which is not colored, clouded, or degenerated even through sterilization treatment, and/or mixtures thereof and preferably includes a cycloolefin copolymer (COC) or a cycloolefin polymer (COP).

At least one of the second sheet 42 and the fourth sheet 44 is preferably black in order to curb reflection of light at the time of microscope observation. For example, the second sheet 42 and the fourth sheet 44 may include a substrate formed of polyethylene terephthalate (PET) and an adhesive having resistance to ethanol on the surface of the substrate. The second sheet 42 may include polyethylene terephthalate (PET) as the substrate. The second sheet 42 may include an adhesive having resistance to ethanol on the surface of the substrate.

The fourth sheet 44 may include polyethylene terephthalate (PET) as the substrate. The fourth sheet 44 may include an adhesive having resistance to ethanol on the surface of the substrate.

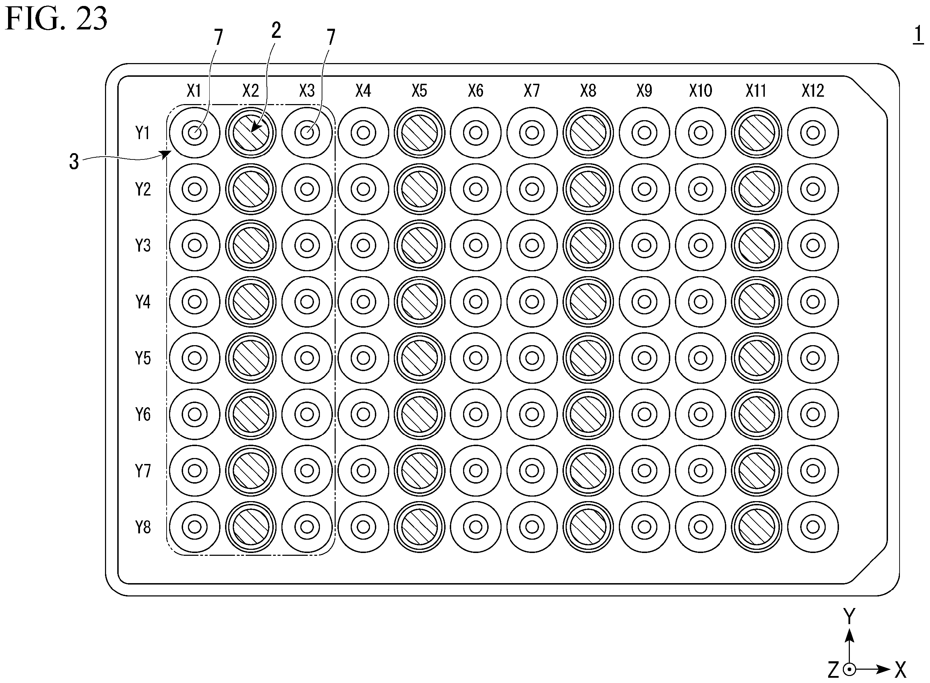

FIG. 23 is a diagram illustrating a case in which 32 sample chambers 2 are provided in a 96 microplate. In the present embodiment, the device 1 has an outer shape of the same size as the microplate in a plan view. 32 sample chambers 2 are provided in the device 1.

For example, four 8-connected units 3 in which the liquid-passing port 7 is disposed at positions X1 and X3 of the microplate and the sample chamber 2 is disposed at a position X2 are arranged in the X direction. Accordingly, it is possible to obtain the device 1 in which the sample chambers 2 are provided at the same positions as wells in columns X2, X5, X8, and X11 of the 96 microplate. By providing the sample chambers 2 at the same positions as in the microplate, it is easy to facilitate automatic observation using a microscope.

For example, an array which four 8-connected units (in 8 units 3 are arranged in the Y direction) are arranged in the X direction may be attached as the flow passage forming members 9, and one flow passage forming member 9 may be attached to the whole device 1. For example, the number of flow passage forming members 9 can be changed according to design specifications.

In the present embodiment, it is possible to perform observation, recovery, culture, and the like of samples (cells or particles) using the device 1.

<Device manufacturing method>

A method of manufacturing the device 1 according to the present embodiment is a device manufacturing method of manufacturing the device 1, the device manufacturing method including a first step of attaching the accommodation layer 5 to the liquid-passing member 8 including the liquid-passing port 7 for allowing the liquid to flow to the accommodation portion 4 and a second step of attaching the flow passage forming member 9 for allowing the liquid to flow between the sample chamber 2, the accommodation layer 5, and the liquid-passing port 7 to the liquid-passing member 8 with the accommodation layer 5 interposed therebetween after the first step.

The first step according to the present embodiment includes an attachment step of attaching the accommodation layer 5 to the first member 10 including the sample chamber 2 and an assembly step of manufacturing the liquid-passing member 8 by assembling the second member 20 including the liquid-passing port 7 to the first member 10 after the attachment step.

For example, in the attachment step of the first step, one film in which the accommodation portion 4 is provided at only a position corresponding to the sample chamber 2 is adhered to the bottom surface of the first member 10 (for example, see FIG. 6). For example, in the assembly step, the first member 10 is assembled from the bottom side of the second member 20 (for example, the arrow direction in FIG. 4). Accordingly, an assembled structure (a coupled structure of the accommodation layer 5 and the liquid-passing member 8) including the accommodation layer 5, the first member 10, and the second member 20 is manufactured.

For example, in the second step, the flow passage forming member 9 is adhered to the bottom surface of the coupled structure. Thereafter, a lid member (not illustrated) covering the sample chamber 2 and the liquid-passing port 7 as a whole is attached to the top of the liquid-passing member 8. As a result, the device 1 according to the present embodiment is manufactured.

<Operations and advantages>

As described above, the device 1 according to the present embodiment is a device including a plurality of units 3 of which each includes a sample chamber 2 that is able to individually accommodate cells or particles. The unit 3 includes the accommodation layer 5 including the accommodation portion 4 corresponding to the sample chamber 2 and the structure 6 allowing a liquid to flow into the accommodation portion 4. The plurality of units 3 are independent such that there is no flow of the liquid therebetween.

With this configuration, since the accommodation portions 4 of the units 3 are independent, a plurality of independent sample chambers 2 can be provided in one device 1. Accordingly, it is possible to independently observe a plurality of samples (cells or particles) in one device 1. For example, it is possible to perform experiment under a plurality of independent conditions in one device.

The structure 6 according to the present embodiment includes the liquid-passing member 8 including the liquid-passing port 7 for allowing the liquid to flow to the accommodation portion 4 and having the accommodation layer 5 attached thereto and the flow passage forming member 9 attached to the liquid-passing member 8 with the accommodation layer 5 interposed therebetween and allowing the liquid to flow between the sample chamber 2, the accommodation layer 5, and the liquid-passing port 7.

With this configuration, it is possible to construct the device 1 using three members (the liquid-passing member 8, the accommodation layer 5, and the flow passage forming member 9).

The liquid-passing member 8 according to the present embodiment may include the first member 10 including the sample chamber 2 and having the accommodation layer 5 attached thereto and the second member 20 including the liquid-passing port 7 and assembled into the first member 10.

With this configuration, since the liquid-passing member 8 is divided into two members (the first member 10 and the second member 20), only the first member 10 is attached to the accommodation layer 5. Since the first member 10 is smaller than the second member 20, the accommodation layers 5 can be formed on the substrate with a higher density when a plurality of accommodation layers 5 corresponding to the first member 10 are formed on one substrate and this is attached to the first member 10 than when the device is constituted using only the three members (the single liquid-passing member, the accommodation layer, and the flow passage forming member). Accordingly, it is possible to greatly increase the number of accommodation layers 5 provided per substrate with the same area.

The flow passage forming member 9 according to the present embodiment includes the first sheet 41 that is transparent, the second sheet 42 that is stacked on the first sheet 41 and includes a communication hole 45 connecting the liquid-passing port 7 to the sample chamber 2, the third sheet 43 that is stacked on the second sheet 42 and includes an opening hole 46 at positions of the liquid-passing member 8 corresponding to the liquid-passing port 7 and the sample chamber 2, and the fourth sheet 44 that is stacked on the third sheet 43 and includes the same shape in a plan view as the third sheet 43. The adhesive layer 40 is provided on both sides of the second sheet 42 and both sides of the fourth sheet 44.

With this configuration, since an adhesive layer 40 may not be provided on both sides of the third sheet 43, it is possible to reduce a contact between the adhesive layer of the third sheet 43 and the flowing liquid as much as possible. For example, since an adhesive layer may not be provided on both sides of the third sheet 43 by employing combination (A) in which an adhesive layer is provided on one side of each of the first sheet 41, the second sheet 42, and the third sheet 43 or combination (B) in which an adhesive layer is provided on both sides of the second sheet 42 and one side of the third sheet 43 out of combinations for providing the adhesive layer, it is possible to achieve this advantageous effect.

For example, when a flow passage is formed using the first sheet 41 that is transparent and does not include an adhesive layer and a sheet including an adhesive layer on both sides thereof, the fourth sheet 44 not including an adhesive layer and having the same shape in a plan view as the third sheet 43 can be inserted between the second sheet 42 and the third sheet 43.

The thickness C1 of the flow passage forming member 9 according to the present embodiment is equal to or less than 2 mm.

With this configuration, it is possible to better facilitate observation with high magnification in comparison with a case in which the thickness C1 of the flow passage forming member 9 is greater than 2 mm.

The accommodation layer 5 according to the present embodiment is a well array filter including a well opening 30 of a size through which a cell or a particle is able to pass. The accommodation layer 5 includes a well bottom 31 corresponding to the well opening 30. The well bottom 31 includes a through-hole 32 through which only the liquid is able to pass.

With this configuration, it is possible to observe a plurality of samples (cells or particles) in one device 1 including a well array filter.

The accommodation layer 5 according to the present embodiment is formed of a negative resist.

With this configuration, it is possible to manufacture the accommodation layer 5 using photolithography (for example, a photoresist application step, an exposure step, and a developing step) which is suitable for micromachining.

The device 1 according to the present embodiment has the same rectangular outer shape as a microplate in a plan view. A sample chambers 2 are provided in the short-side direction of the device 1. B sample chambers 2 are provided in the long-side direction of the device 1. The ratio A:B of the number of sample chambers 2 A arranged in the short-side direction in the device 1 to the number of sample chambers 2 B arranged in the long-side direction is 2:1.

With this configuration, when the ratio A:B is 2: 1, an arrangement of two liquid-passing ports + one sample chamber (when 32 sample chambers 2 are provided in a 96 microplate) can be provided at the same positions as three wells adjacent to each other vertically or horizontally. For example, a sample chamber 2 and two liquid-passing ports 7 are preferably provided such that the sample chamber 2 is interposed between the two liquid-passing ports 7.

The device 1 according to the present embodiment has an outer shape of the same size as a microplate in a plan view. 32 sample chambers 2 are provided in the device 1.

With this configuration, it is possible to obtain a device 1 of the same size as a microplate in a plan view.

An observation method according to the present embodiment is an observation method of individually observing the cells or the particles using the device 1 and a microscope.

With this method, since the device 1 is used, it is possible to independently observe a plurality of samples (cells or particles) in one device 1.

The observation method may use the device 1. The observation method may be an observation method of individually observing the cells or the particles using a microscope.

A recovery method according to the present embodiment is a recovery method of individually recovering the cells or the particles using the device 1.

With this method, since the device 1 is used, it is possible to independently recover a plurality of samples (cells or particles) in one device 1.

The recovery method may use the device 1. The recovery method may be a recovery method of individually recovering the cells or the particles.

A culture method according to the present embodiment is a culture method of culturing the cells using the device 1.

With this method, since the device 1 is used, it is possible to independently culture a plurality of samples (cells) in one device 1.

The culture method may use the device 1. The culture method may be a culture method of culturing the cells.

A method of manufacturing the device 1 according to the present embodiment is a device manufacturing method of manufacturing the device 1, the device manufacturing method including a first step of attaching the accommodation layer 5 to the liquid-passing member 8 including the liquid-passing port 7 for allowing the liquid to flow to the accommodation portion 4 and a second step of attaching the flow passage forming member 9 for allowing the liquid to flow between the sample chamber 2, the accommodation layer 5, and the liquid-passing port 7 to the liquid-passing member 8 with the accommodation layer 5 interposed therebetween after the first step.

With this method, it is possible to obtain a device 1 including three members (the liquid-passing member 8, the accommodation layer 5, and the flow passage forming member 9) through two steps (the first step and the second step).

The device 1 manufacturing method may be a device manufacturing method of manufacturing the device 1. The device 1 manufacturing method may include the first step of attaching the accommodation layer 5 to the liquid-passing member 8 including a liquid-passing port 7 for allowing the liquid to flow to the accommodation portion 4. The device 1 manufacturing method may include a second step of attaching the flow passage forming member 9 for allowing the liquid to flow between the sample chamber 2, the accommodation layer 5, and the liquid-passing port 7 to the liquid-passing member 8 with the accommodation layer 5 interposed therebetween after the first step.

The first step according to the present embodiment includes an attachment step of attaching the accommodation layer 5 to the first member 10 including the sample chamber 2 and an assembly step of manufacturing the liquid-passing member 8 by assembling the second member 20 including the liquid-passing port 7 to the first member 10 after the attachment step.

With this method, since the liquid-passing member 8 is divided into two members (the first member 10 and the second member 20), the accommodation layer 5 is attached to only the first member 10 in the attachment step. Accordingly, when a member in which individual accommodation layers are manufactured on different substrates or a connected member in which individual accommodation layers are connected is used, it is possible to use a member in which accommodation layers are integrated with a high density in comparison with a case in which two steps (the first step and the second step) are simply used. As a result, it is possible to greatly increase the number of substrates or connected members provided per unit area.

The first step may include an attachment step of attaching the accommodation layer 5 to the first member 10 including the sample chamber 2. The first step may include an assembly step of manufacturing the liquid-passing member 8 by assembling the second member 20 including the liquid-passing port 7 to the first member 10 after the attachment step.

<Second embodiment>

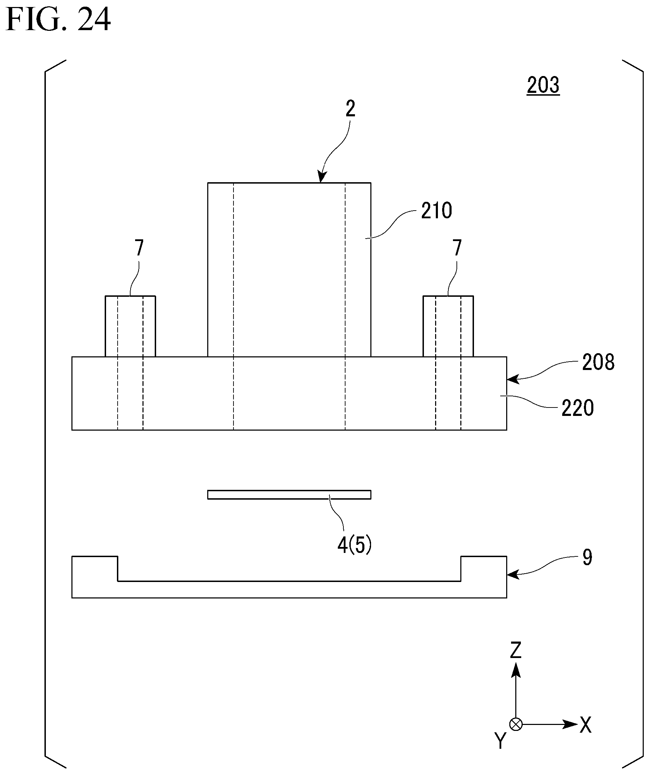

FIG. 24 is an exploded view of a unit 203 according to a second embodiment.

**In** the first embodiment, an example in which the liquid-passing member 208 is divided into two members (the first member 10 and the second member 20) has been described above, and the present invention is not limited thereto. For example, as illustrated in FIG. 24, the liquid-passing member 8 may not be divided into two members (the first member 10 and the second member 20). In the second embodiment, the same constituents as in the first embodiment will be referred to by the same reference signs, and detailed description thereof will be omitted.

The liquid-passing member 208 according to the present embodiment includes a first liquid-passing portion 210 that includes a sample chamber 2 and is attached to the accommodation layer 5 and a second liquid-passing portion 220 including a liquid-passing port 7. In the present embodiment, the liquid-passing member 208 is a unified object. For example, the first liquid-passing portion 210 and the second liquid-passing portion 220 may be formed as a unified body out of the same member.

<Operations and advantages>

As described above, the liquid-passing member 208 according to the present embodiment is a unified object.

With this configuration, it is possible to reduce the number of components in comparison with a case in which the liquid-passing member 8 is divided into two members (the first member 10 and the second member 20).

<Third embodiment>

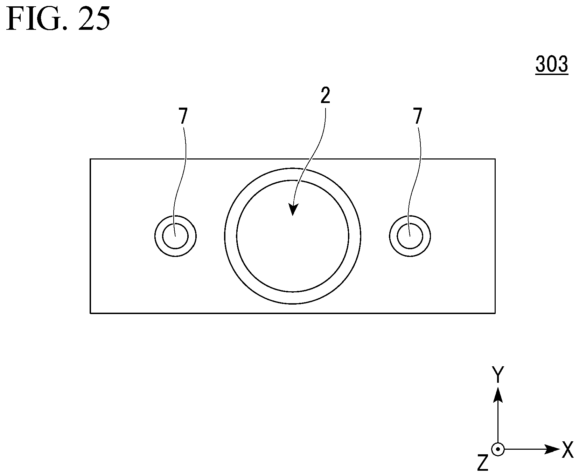

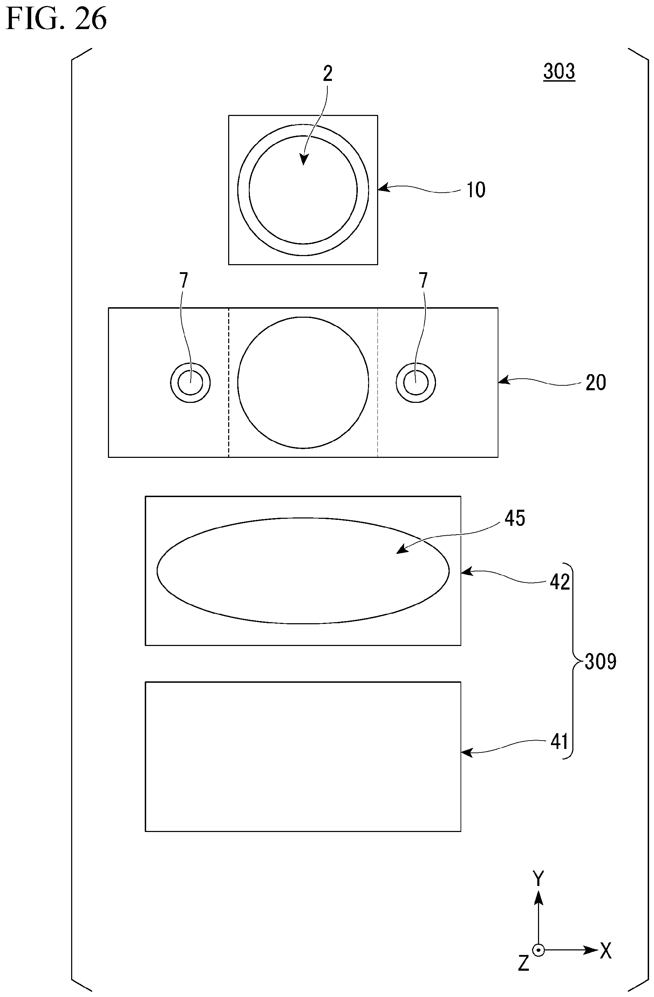

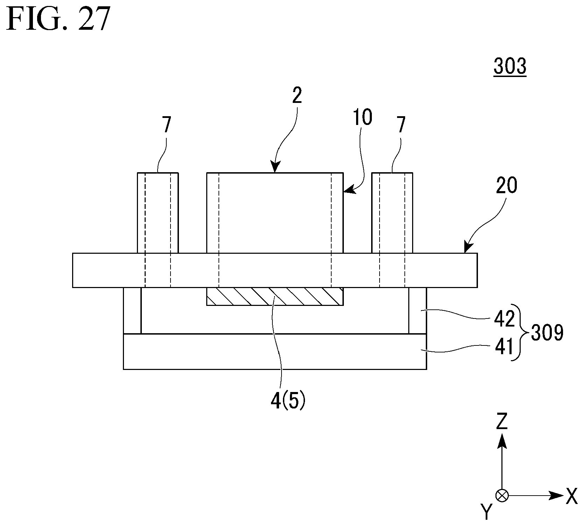

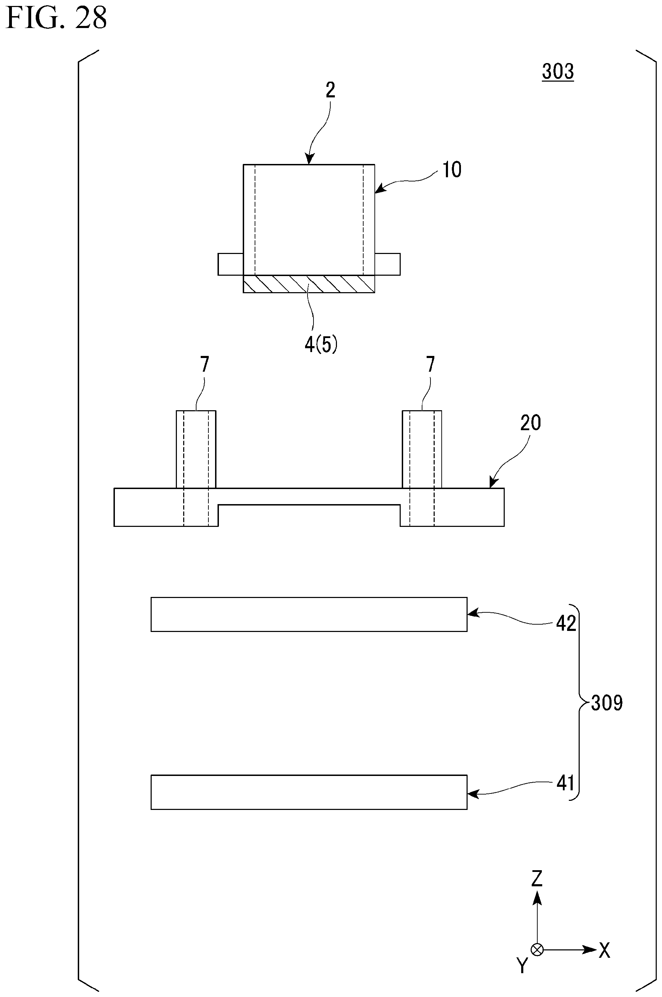

FIG. 25 is a top view of a unit 303 according to a third embodiment. FIG. 26 is an exploded top view of the unit 303 according to the third embodiment. FIG. 27 is a side view of the unit 303 according to the third embodiment. FIG. 28 is an exploded side view of the unit 303 according to the third embodiment.

In the first embodiment, an example in which the flow passage forming member 9 includes four sheets (the first sheet 41, the second sheet 42, the third sheet 43, and the fourth sheet 44) has been described above, and the present invention is not limited thereto. For example, as illustrated in FIGS. 25 to 28, a flow passage forming member 309 may not include the third sheet 43 and the fourth sheet 44. In the third embodiment, the same constituents as in the first embodiment will be referred to by the same reference signs, and detailed description thereof will be omitted.

The flow passage forming member 309 according to the present embodiment includes a first sheet 41 that is transparent and a second sheet 42 that is stacked on the first sheet 41 and includes a communication hole 45 connecting the liquid-passing port 7 to the sample chamber 2. The flow passage forming member 309 can be manufactured by stacking the first sheet 41 having no hole and the second sheet 42 including the communication hole 45 connecting the liquid-passing port 7 to the sample chamber 2 without including the third sheet 43 and the fourth sheet 44.

An adhesive layer 40 is provided on both sides of the second sheet 42. The color of the second sheet 42 is preferably black in order to curb reflection of light at the time of microscope observation. For example, the second sheet 42 may include polyethylene terephthalate (PET) as a substrate and include an adhesive having resistance to ethanol on the surface of the substrate.

<Operations and advantages>

As described above, the flow passage forming member 309 according to the present embodiment includes the first sheet 41 that is transparent and the second sheet 42 that is stacked on the first sheet 41 and includes a communication hole 45 connecting the liquid-passing port 7 to the sample chamber 2.

With this configuration, it is possible to obtain the flow passage forming member 309 when one sample chamber 2 is provided in one unit 303. For example, an adhesive layer may be provided on both sides of the second sheet 42, or an adhesive layer may be provided on one side of each of the first sheet 41 and the second sheet 42 in addition.

<Fourth embodiment>

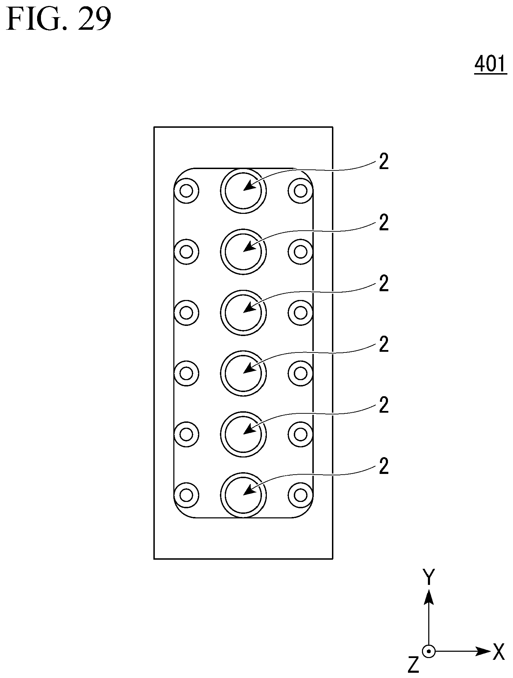

FIG. 29 is a top view of a device 401 according to a fourth embodiment.

In the first embodiment, an example in which the device 1 has the same rectangular outer shape of a microplate in a plan view has been described above, and the present invention is not limited thereto. For example, as illustrated in FIG. 29, the device 401 may have an outer shape of the same size as slide glass in a plan view. In the fourth embodiment, the same constituents as in the first embodiment will be referred to by the same reference signs, and detailed description thereof will be omitted.

The slide glass is a glass plate that is mainly used to load a minute sample at the time of observation using an optical microscope. In general, as a size in a plan view of the slide glass, a length in the long-side direction (the Y direction) (an outer dimension of the long side) is about 75 mm, and a length in the short-side direction (the X direction) (an outer dimension of the short side) is about 25 mm.

In the illustrated example (the top view of FG. 29), six sample chambers 2 are provided in the device 401. The number of sample chambers 2 is not limited thereto, and two, four, or eight sample chambers may be provided in the device 401. For example, the shape in a plan view of the device 401 and the number of sample chambers 2 to be installed can be changed according to design specifications.

<Operations and advantages>

As described above, the device 401 according to the present embodiment has an outer shape of the same size as the slide glass in a plan view. Six sample chambers 2 are provided in the device 401.

With this configuration, it is possible to obtain the device 401 of the same size as the slide glass in a plan view.

<Fifth embodiment>

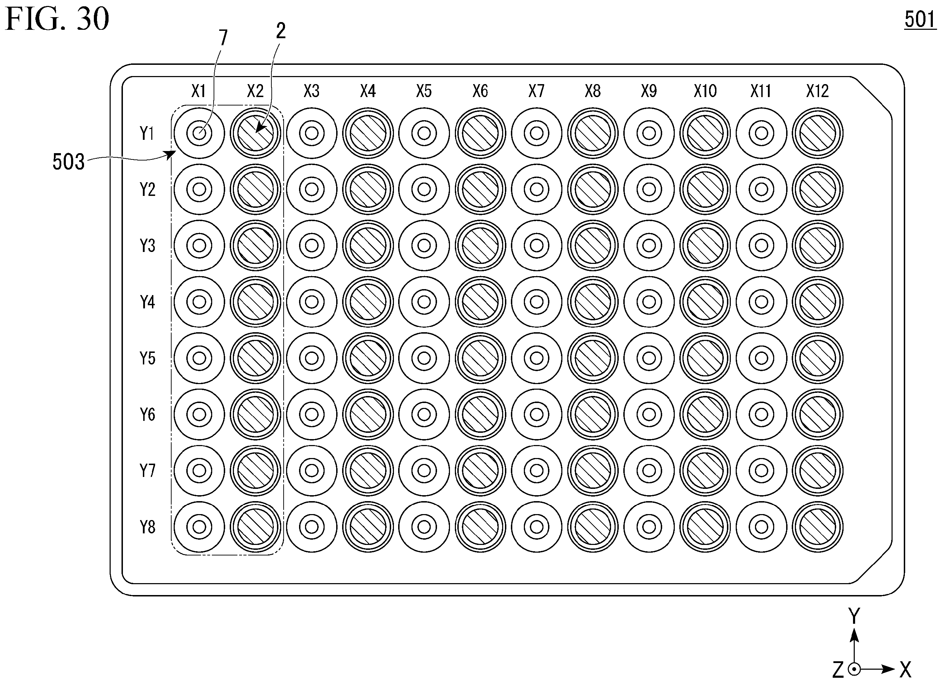

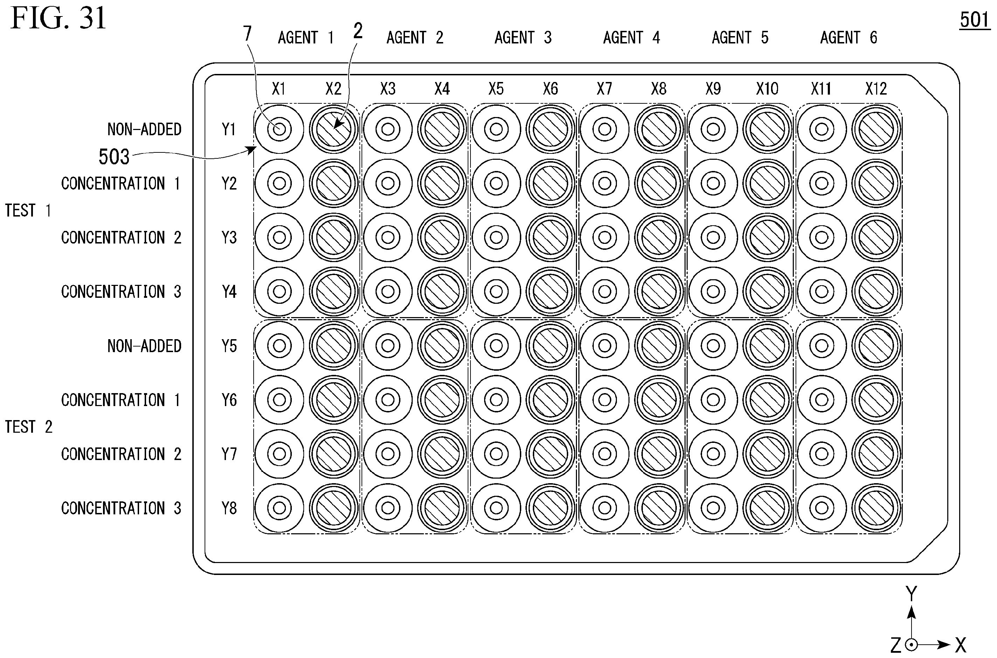

FIG. 30 is a top view of a device 501 according to a fifth embodiment. FIG. 31 is a diagram illustrating an experimental example using the device 501 according to the fifth embodiment.

In the first embodiment, an example in which the ratio A:B of the number of sample chambers 2 A arranged in the short-side direction (the Y direction) in the device 1 to the number of sample chambers 2 B arranged in the long-side direction (the X direction) is 2:1 has been described above, and the present invention is not limited thereto. For example, as illustrated in FIG. 30, the ratio A:B of the number of sample chambers 2 A arranged in the short-side direction in the device 501 to the number of sample chambers 2 B arranged in the long-side direction may be 2:1.5.

Referring to FIGS. 30 and 31 together, according to the present embodiment, the number of sample chambers 2 A arranged in the short-side direction in the device 501 is eight, and the number of sample chambers 2 B arranged in the long-side direction is six. 48 sample chambers 2 are provided in the device 501. The number of sample chambers 2 provided in the device 501 is not limited thereto and can be changed according to design specifications.

In the device 501 according to the present embodiment, six 8-connected units 503 in which the liquid-passing port 7 is arranged on only one side of the sample chamber 2 are arranged in the X direction. For example, six 8-connected units 3 in which the liquid-passing ports 7 are provided at a position X1 of a microplate and the sample chambers 2 are arranged at a position X2 are provided in the X direction. Accordingly, it is possible to obtain the device 501 in which the sample chambers 2 are provided at the same positions as the wells in columns X2, X4, X6, X8, X10, and X12 of a 96 microplate. By providing the sample chambers 2 at the same positions as in the microplate in this way, automatic observation using a microscope is facilitated.

When the device 501 includes 48 sample chambers 2 in this way, the agent response of cells can be checked at a single-cell level. For example, six types of agent responses (for example, responses of agents 1 to 6) can be checked to correspond to two tests (for example, test 1 and test 2) under four concentration conditions (for example, four conditions including non-added, concentration 1, concentration 2, and concentration 3).

<Operations and advantages>

As described above, the device 501 according to the present embodiment has the same rectangular outer shape as the microplate in a plan view. A sample chambers 2 are arranged in the short-side direction of the device 501, and B sample chambers 2 are arranged in the long-side direction of the device 501. The ratio A:B of the number of sample chambers 2 A arranged in the short-side direction in the device 501 to the number of sample chambers 2 B arranged in the long-side direction is 2:1.5.

With this configuration, since the ratio A:B is 2:1.5, an array of one liquid-passing port and one sample chamber (when 48 sample chambers 2 are provided in the 96 microplate) can be provided at the same positions as two neighboring wells in the microplate.

<Modified examples>

In the aforementioned embodiments, an example in which the structure includes the liquid-passing member including the liquid-passing port allowing a liquid to flow to the accommodation portion and having the accommodation layer attached thereto and the flow passage forming member attached to the liquid-passing member via the accommodation layer and allowing a liquid to flow between the sample chamber, the accommodation layer, and the liquid-passing port has been described above, and the present invention is not limited thereto. For example, the structure has only to have a structure for allowing a liquid to flow to the accommodation portion. For example, the device may be constructed using only two members (the accommodation layer and the structure). For example, the configuration of the structure can be changed according to design specifications.

In the aforementioned embodiments, an example in which the liquid-passing member includes the first member including a sample chamber and having the accommodation layer attached thereto and the second member including a liquid-passing port and attached to the first member has been described above, and the present invention is not limited thereto. For example, the liquid-passing member may not be divided into two members (the first member and the second member). For example, the liquid-passing member may include only one member. For example, the configuration of the liquid-passing member can be changed according to design specifications.

In the aforementioned embodiments, an example in which the thickness of the flow passage forming member is equal to or less than 2 mm has been described above, and the present invention is not limited thereto. For example, the thickness of the flow passage forming member may be greater than 2 mm. For example, the thickness of the flow passage forming member can be changed according to design specifications.

In the aforementioned embodiments, the accommodation layer is a well array filter including a well opening of a size through which a cell or a particle is able to pass and includes a well bottom corresponding to the well opening. An example in which the well bottom includes a through-hole through which only a liquid can be allowed to pass has been described above, and the present invention is not limited thereto. For example, the well bottom may include a through-hole through which only a cell or a particle smaller than a specific size is allowed to pass. For example, the through-hole may be configured to allow a sample other than a liquid to pass therethrough. For example, the configuration of the through-hole in the well bottom can be changed according to design specifications.

In the aforementioned embodiments, an example in which the accommodation layer is formed of a negative resist has been described above, and the present invention is not limited thereto. For example, the accommodation layer may be formed of a positive resist. For example, the material of the accommodation layer can be changed according to design specifications.

In the aforementioned embodiments, an example in which the ratio A:B of the number of sample chambers A arranged in the short-side direction in the structure to the number of sample chambers B arranged in the long-side direction is 2:1.5 or 2:1 has been described above, and the present invention is not limited thereto. For example, the ratio A:B may be 2:3. When the ratio A:B is 2:3, the sample chambers can be provided at the same positions as wells in the microplate which is commercially available. For example, vertical and horizontal pitches between the sample chambers can be the same similarly to the wells in the microplate. For example, the structure may not have the same rectangular outer shape as the microplate in a plan view. For example, the ratio A:B may be a ratio other than (2:3, 2:1.5, or 2:1). For example, the shape in a plan view of the structure, the ratio A:B, and the number of sample chambers can be changed according to design specifications.

In the aforementioned embodiments, an example in which the flow passage forming member includes four sheets (the first embodiment) or two sheets (the third embodiment) has been described above, and the present invention is not limited thereto. For example, the flow passage forming member may include three sheets. For example, the flow passage forming member may include a first sheet that is transparent, a second sheet that is stacked on the first sheet and includes a communication hole connecting the liquid-passing port to the sample chamber, and a third sheet that is stacked on the second sheet and includes opening holes at positions of the liquid-passing member corresponding to the liquid-passing port and the sample chamber. With this configuration, it is possible to obtain the flow passage forming member when a plurality of sample chambers are provided in one unit. For example, it is possible to cause the sheets to adhere by providing an adhesive layer between the sheets. There are three following combinations associated with in what sheet an adhesive layer is to be provided.

(A) An adhesive layer may be provided on one side of each of the first sheet, the second sheet, and the third sheet.
(B) An adhesive layer may be provided on both sides of the second sheet and one side of the third sheet.
(C) An adhesive layer may be provided on one side of the second sheet and both sides of the third sheet.

For example, the number of sheets included in the flow passage forming member can be changed according to design specifications.

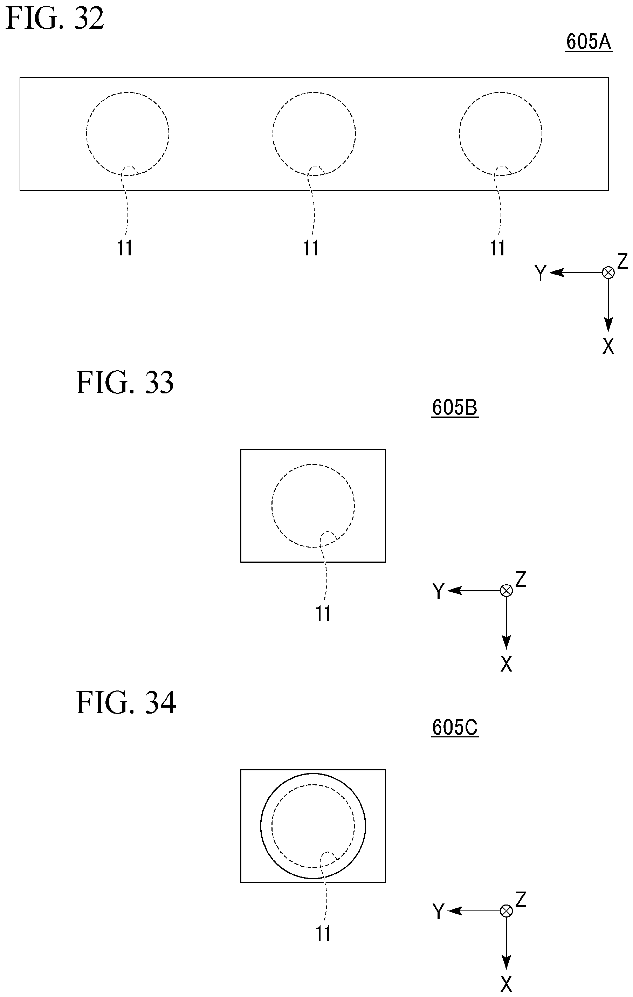

In the aforementioned embodiments, an example in which one film in which the accommodation portion is provided at only the positions corresponding to the sample chambers is attached to the bottom surface of the first member has been described above, and the present invention is not limited thereto. FIG. 32 is a bottom view of an accommodation layer according to a first modified example. FIG. 33 is a bottom view of an accommodation layer according to a second modified example. FIG. 34 is a bottom view of an accommodation layer according to a third modified example. For example, as illustrated in FIG. 32, a film 605A constituting the accommodation layer may be a film which is formed as an accommodation portion as a whole. For example, as illustrated in FIG. 33, a film 605B constituting the accommodation layer may be a film in which an accommodation portion is provided in only a lower part of the sample chamber opening 11 of the first member. For example, as illustrated in FIG. 34, in a film 605C constituting the accommodation layer, the shape in a plan view of the accommodation portion may be a circular shape larger than the shape (circular shape) in a plan view of the bottom of the sample chamber opening 11. For example, the shape in a plan view and the size of the accommodation layer, the arrangement and configuration of the accommodation portion, and the like can be changed according to design specifications.

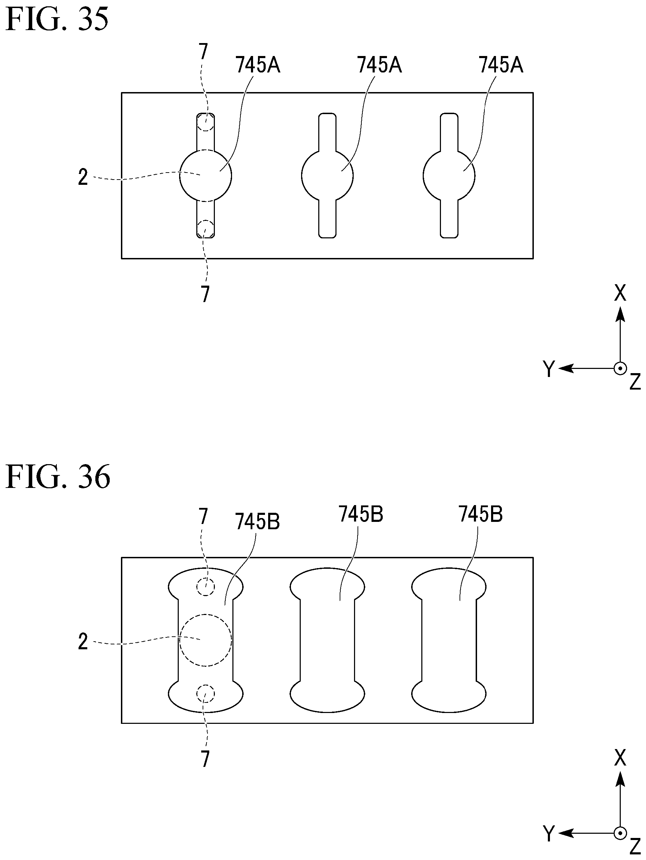

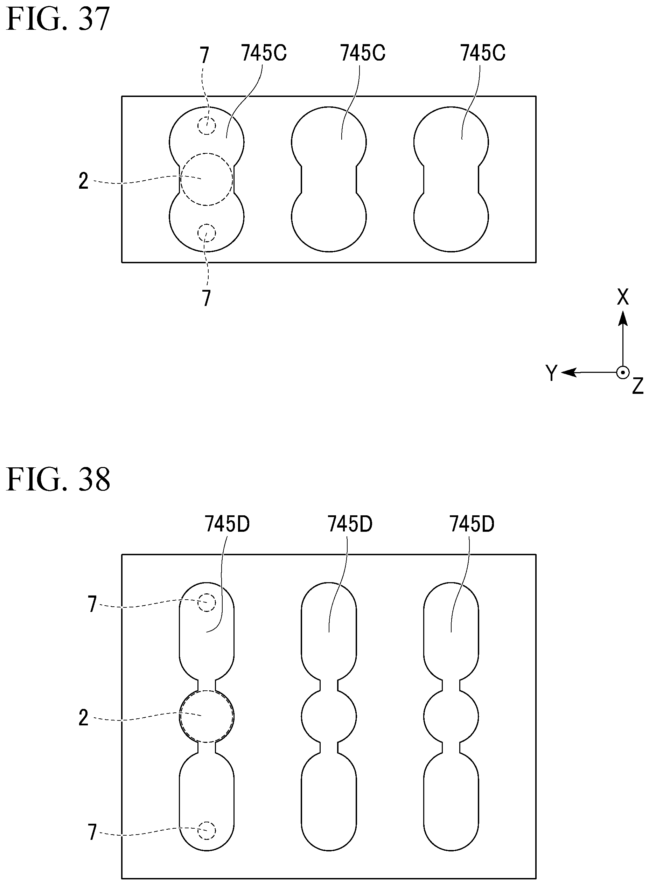

In the aforementioned embodiments, an example in which the shape in a plan view of a communication hole of the second sheet in the flow passage forming member is an elliptical shape surrounding the openings of the sample chamber and the liquid-passing port has been described above, and the present invention is not limited thereto. FIG. 35 is a diagram illustrating a shape of a communication hole 745A in a flow passage forming member according to a fourth modified example. FIG. 36 is a diagram illustrating a shape of a communication hole 745B in a flow passage forming member according to a fifth modified example. FIG. 37 is a diagram illustrating a shape of a communication hole 745C in a flow passage forming member according to a sixth modified example. FIG. 38 is a diagram illustrating a shape of a communication hole 745D in a flow passage forming member according to a seventh modified example. For example, as illustrated in FIG. 35, the shape in a plan view of the communication hole 745A of the second sheet in the flow passage forming member may be a shape in which the circular shape surrounding the opening of the sample chamber 2 and the rectangular shape surrounding the opening of the liquid-passing port 7 are combined. With this configuration, since the shape of the communication hole 745A of the second sheet is a shape in which the width on the liquid-passing port 7 side is smaller than the width on the sample chamber 2 side in a plan view, it is possible to minimize the volume of a flow passage. For example, as illustrated in FIG. 36, the shape in a plan view of the communication hole 745B of the second sheet in the flow passage forming member may be a shape in which the rectangular shape surrounding the opening of the sample chamber 2 and the elliptical shape surrounding the opening of the liquid-passing port 7 are combined. With this configuration, since the shape of the communication hole 745B of the second sheet is a shape in which the width on the liquid-passing port 7 side is larger than the width on the sample chamber 2 side in a plan view, it is possible to decrease a pressure required for suction of a liquid from the liquid-passing port 7 in comparison with a case in the shape illustrated in FIG. 35. For example, as illustrated in FIG. 37, the shape in a plan view of the communication hole 745C of the second sheet in the flow passage forming member may be a shape in which the rectangular shape surrounding the opening of the sample chamber 2 and the circular shape surrounding the opening of the liquid-passing port 7 are combined. With this configuration, since the shape of the communication hole 745C of the second sheet is a shape in which the width on the liquid-passing port 7 side is larger than the width on the sample chamber 2 side in a plan view similarly to the shape illustrated in FIG. 36, it is possible to decrease a pressure required for suction of a liquid from the liquid-passing port 7. For example, as illustrated in FIG. 38, the shape in a plan view of the communication hole 745D of the second sheet in the flow passage forming member may be a shape in which the circular shape surrounding the opening of the sample chamber 2 and the elliptical shape surrounding the opening of the liquid-passing port 7 are combined. With this configuration, since the shape of the communication hole 745D of the second sheet can increase the pressure required for suction of a liquid from the liquid-passing port 7 and sufficiently increase the volume on the liquid-passing port 7 side similarly to the shape illustrated in FIG. 35, it is possible to more easily move bubbles hindering observation from the sample chamber 2 to the flow passage. The shape of the communication hole of the second sheet may be a shape in which the width on the liquid-passing port side is smaller than the width on the sample chamber side in a plan view or a shape in which the width on the liquid-passing port side is larger than the width on the sample chamber side in a plan view. For example, the shape in a plan view of the communication hole of the second sheet can be changed according to design specifications.

In the present embodiments, samples (cells or particles) which are observed, recovered, cultured, and the like are not particularly limited. For example, when cells are used as the sample, the sample may include only a single cell or a group of a plurality of cells. For example, a cell includes a cell mass (a cell aggregation).

<Sixth embodiment>

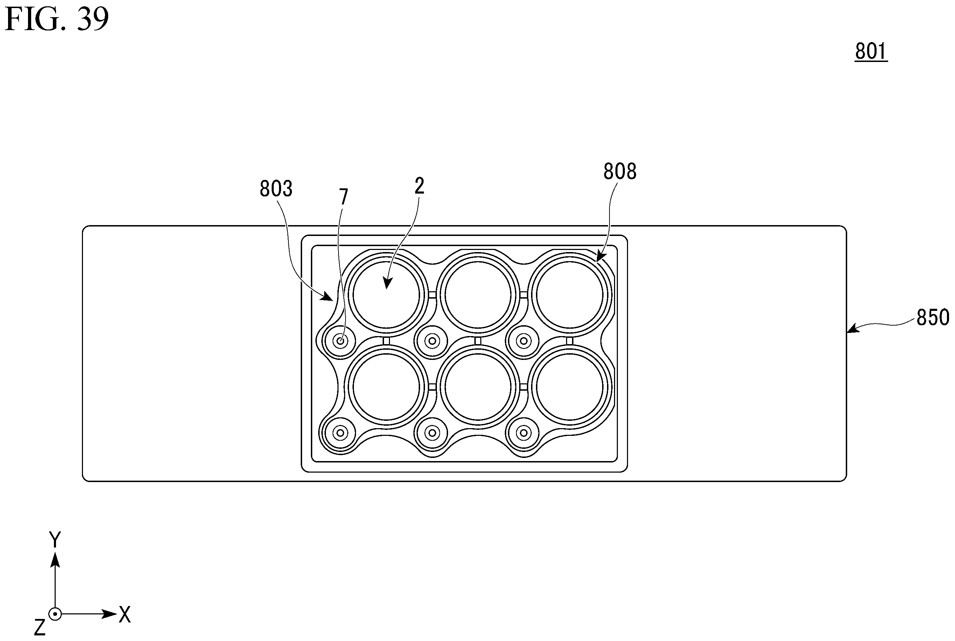

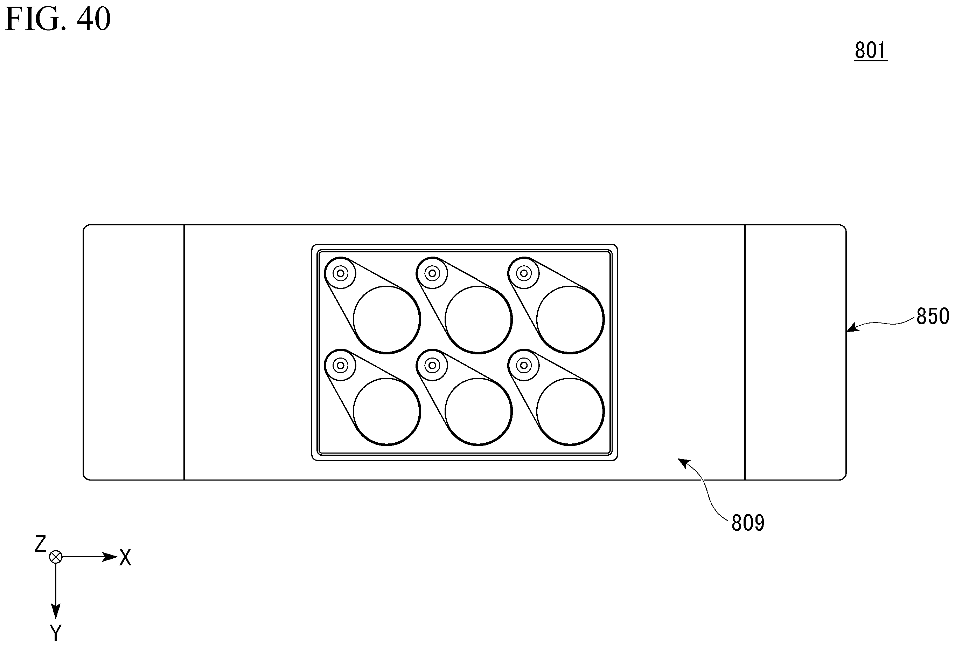

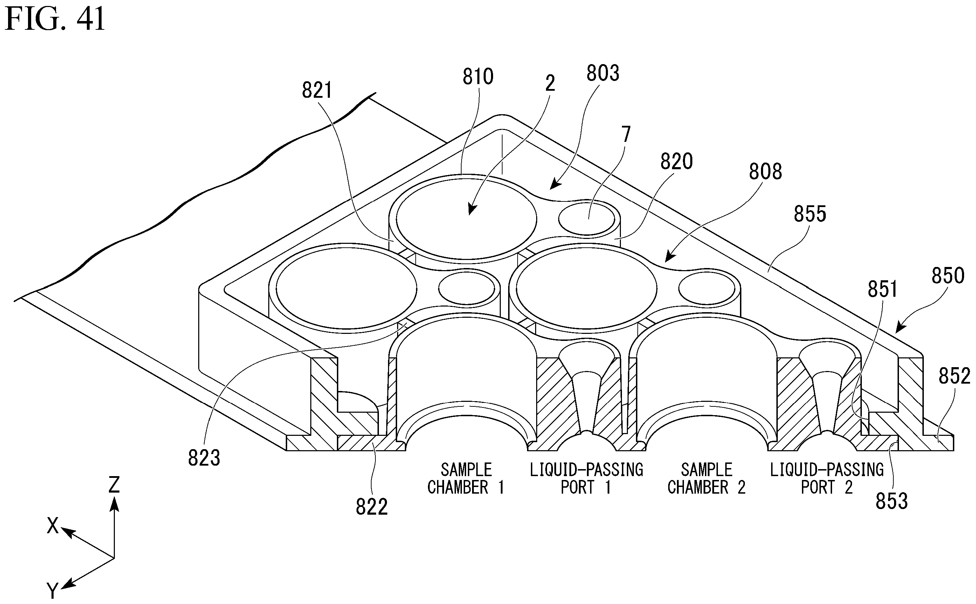

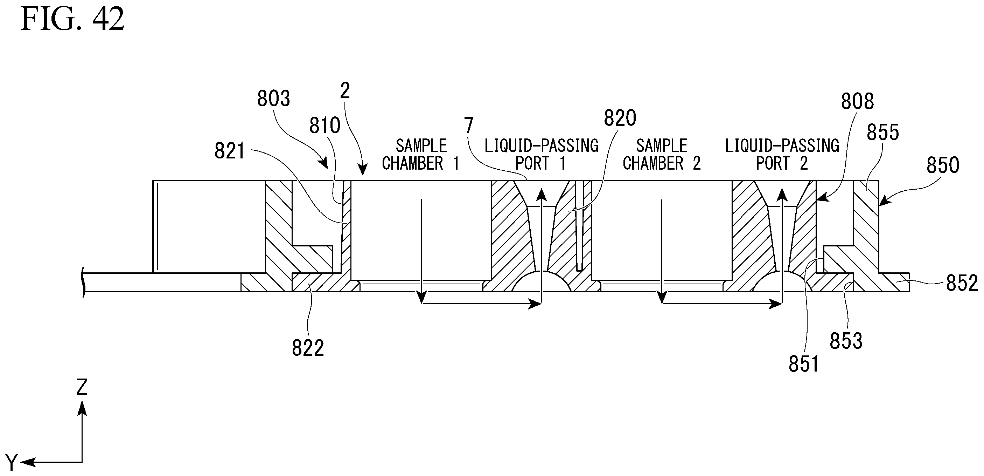

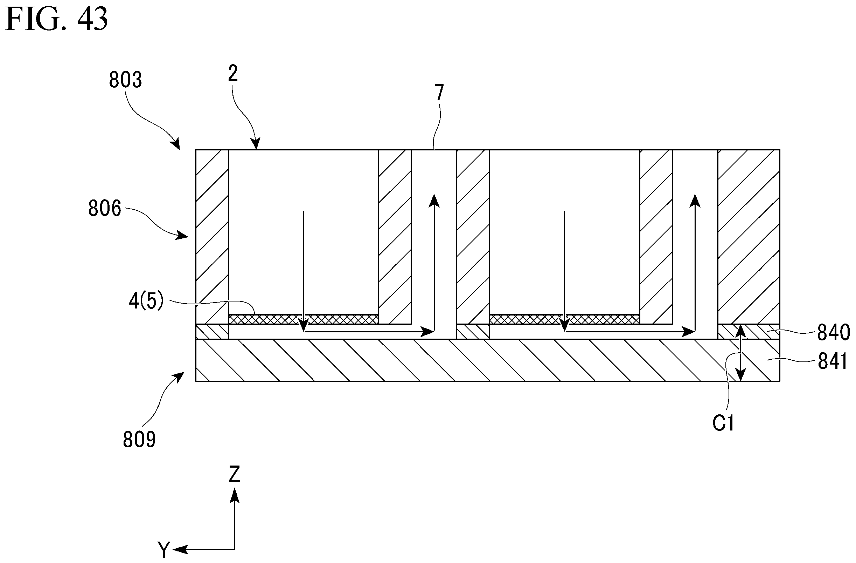

FIG. 39 is a top view of a device 801 according to a sixth embodiment. FIG. 40 is a bottom view of the device 801 according to the sixth embodiment. FIG. 41 is a perspective view including a cross-section of a body 808 and a base 850 according to the sixth embodiment. FIG. 42 is a sectional view of the body 808 and the base 850 according to the sixth embodiment. FIG. 43 is a diagram illustrating a flow of a liquid in the device 801 according to the sixth embodiment.

In the sixth embodiment, the same constituents as in the aforementioned embodiments will be referred to by the same reference signs, and detailed description thereof will be omitted.

As illustrated in FIGS. 39 to 43, the device 801 includes a plurality of units 803 of which each includes a sample chamber 2 capable of individually accommodating samples (cells or particles). The device 801 may have an outer shape of the same size as slide glass in a plan view.

Each unit 803 includes an accommodation layer 5 including an accommodation portion 4 corresponding to the sample chamber 2 and a structure 806 allowing a liquid to flow to the accommodation portion 4. The plurality of units 803 are independent such that there is no flow of a liquid therebetween. In FIGS. 42 and 43, arrows indicate flow directions of a liquid in the device 801 (each unit 803).

The device 801 includes a body 808 (corresponding to the liquid-passing member) including the sample chamber 2 and the liquid-passing port 7, a base 850 that is assembled into the body 808, and a flow passage forming member 809 that allows a liquid to flow between the sample chamber 2, the accommodation layer 5, and the liquid-passing port 7.

For example, since a distance from the bottom of the device 801 to the accommodation layer 5 can be decreased as the thickness C1 of the flow passage forming member 809 decreases, high-magnification observation is facilitated. In the present embodiment, it is possible to easily decrease the thickness C1 of the flow passage forming member 809 in comparison with a structure in which the sample chamber and the liquid-passing port are divided.

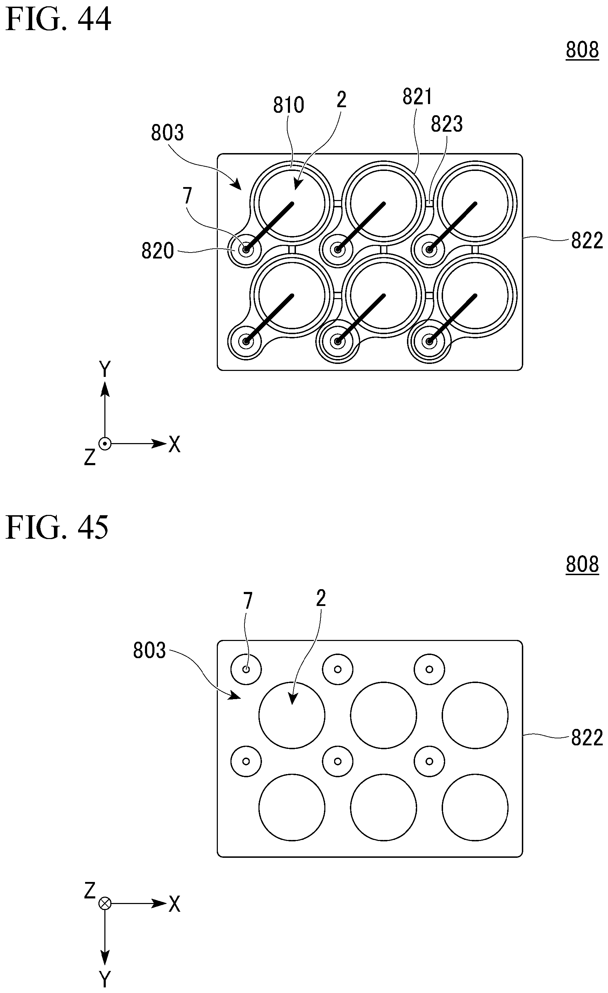

FIG. 44 is a top view of the body 808 according to the sixth embodiment. FIG. 45 is a bottom view of the body 808 according to the sixth embodiment. In FIG. 44, a combination of one sample chamber 2 and one liquid-passing port 7 is indicated by a bold line.

Referring to FIGS. 44 and 45 together, the body 808 includes the sample chamber 2 and the liquid-passing port 7. The body 808 includes a first tubular portion 810 in which the sample chamber 2 is formed and a second tubular portion 820 in which the liquid-passing port 7 is formed. The first tubular portion 810 and the second tubular portion 820 are connected obliquely with respect to the X direction and the Y direction.

A sample chambers 2 are provided in the short-side direction (the Y direction) of the device 801, and B sample chambers 2 are provided in the long-side direction (the X direction) of the device 801. In the present embodiment, the ratio A:B of the number of sample chambers 2 A arranged in the short-side direction in the device 801 to the number of sample chambers 2 B arranged in the long-side direction is 2:3. The ratio A:B is not limited thereto and can be changed according to design specifications.

In the present embodiment, the number of sample chambers 2 A arranged in the short-side direction in the device 801 is two, and the number of sample chambers 2 B arranged in the long-side direction is three. Six sample chambers 2 are provided in the device 801. The number of sample chambers 2 provided in the device 801 is not limited thereto and can be changed according to design specifications.

In the present embodiment, the same number of liquid-passing ports 7 as the number of sample chambers 2 is provided at the positions corresponding to the sample chambers 2. Each liquid-passing port 7 is disposed at a position at which the slopes in the -X direction and the -Y direction are offset from the sample chamber 2 at the corresponding position. Six liquid-passing ports 7 are provided in the device 801. The arrangement and the number of the liquid-passing ports 7 provided in the device 801 are not limited thereto and can be changed according to design specifications.

In the present embodiment, the shape of the upper part of the liquid-passing port 7 is an inverted conical shape (a shape of which the diameter increases upward). The shape of the lower part of the liquid-passing port 7 is a dome shape (a semi-spherical shape which is curved upward). As a result, bubbles are less likely to gather in the flow passage.

The material of the body 808 can be arbitrarily selected according to a subject accommodated in the sample chambers 2. The body 808 can additionally include one or more materials selected from a group consisting of a material not having cytotoxicity, a material in which cytotoxic components are not eluted, a material having low autofluorescence, a material which is not significantly degraded by gamma irradiation, a material which is not significantly degraded by ethylene oxide steam exposure, a material which is not significantly degraded by ultraviolet irradiation, a material which is not significantly degraded by sterilization treatment, a material which does not dissolve in alcohol, and/or mixtures thereof and preferably includes a material which does not have cytotoxicity and in which cytotoxic components are not eluted.

The color of the body 808 can be arbitrarily selected according to a subject accommodated in the sample chambers 2. The color of the body 808 is preferably black in view of reduction of autofluorescence of the material and an influence of reflection of light at the time of microscope observation.

The body 808 according to the present embodiment includes a communication wall portion 821 including the sample chamber 2 and the liquid-passing port 7, a platelike substrate portion 822 connected to the communication wall portion 821, and a connection portion 823 connecting neighboring communication wall portions 821. The body 808 is formed as one component. For example, the communication wall portion 821, the substrate portion 822, and the connection portion 823 may be formed as a unified body out of the same member. The body 808 has a structure in which six communication wall portions 821 are connected on the substrate portion 822. The number of communication wall portions 821 provided on the substrate portion 822 is not limited thereto and can be changed according to design specifications.

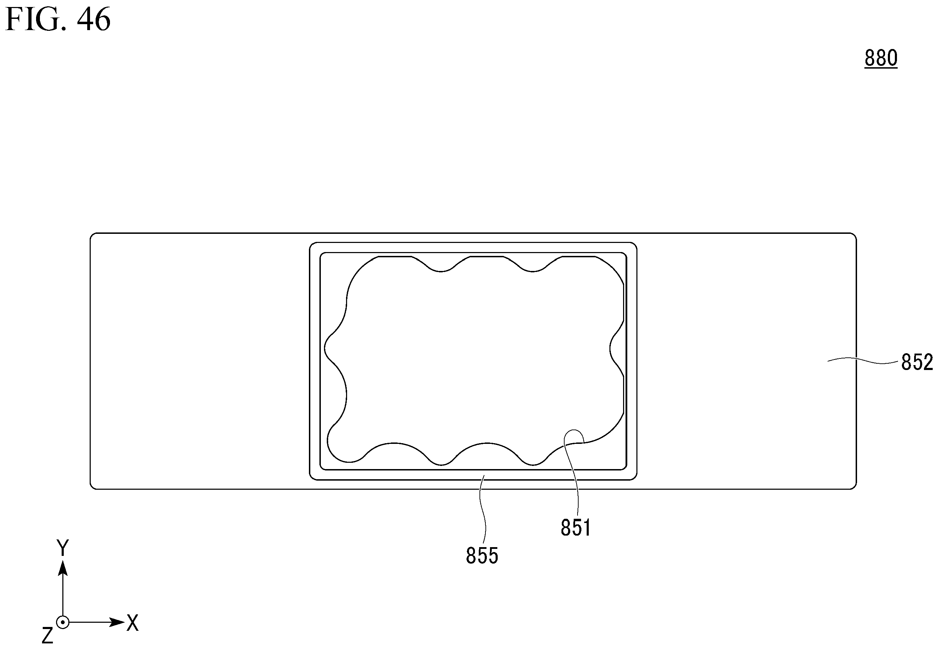

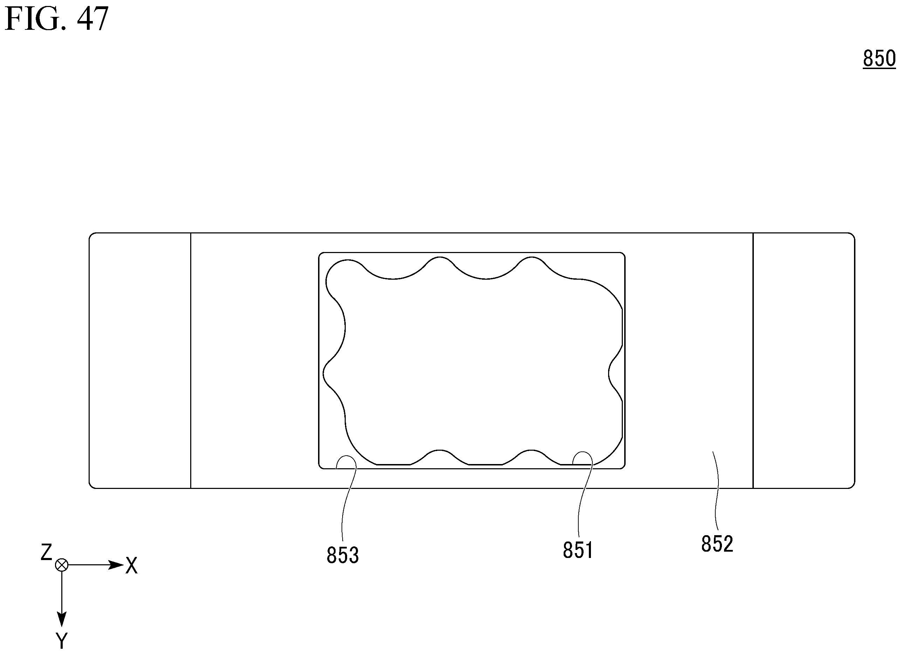

FIG. 46 is a top view of the base 850 according to the sixth embodiment. FIG. 47 is a bottom view of the base 850 according to the sixth embodiment.

Referring to FIGS. 46 and 47 together, the base 850 is a member to which the body 808 is assembled.

The base 850 includes a bottom wall portion 852 including an opening hole 851 into which the body 808 is inserted. The bottom wall portion 852 includes a recessed portion 853 into which the substrate portion 822 of the body 808 is fitted on the bottom surface thereof. The opening hole 851 of the bottom wall portion 852 is formed along the outer shape of the plurality of communication wall portion 821 of the body 808. The outer shape of the recessed portion 853 of the bottom wall portion 852 is a rectangular shape in a plan view such that the substrate portion 822 of the body 808 is fitted thereinto.

For example, when the body 808 and the base 850 are assembled, the body 808 is inserted into the opening hole 851 of the bottom wall portion 852 of the base 850 from the bottom side of the base 850. Accordingly, the communication wall portion 821 of the body 808 is inserted into the opening hole 851 of the bottom wall portion 852 of the base 850, and the substrate portion 822 of the body 808 is fitted into the recessed portion 853 of the bottom wall portion 852. For example, the bottom surfaces of the body 808 and the base 850 which have been assembled are flush with each other.

The base 850 includes a rectangular frame wall portion 855 including rounded corners in a plan view on the top surface of the bottom wall portion 852. The frame wall portion 855 is provided to surround all of the plurality of communication wall portions 821 (all the sample chambers 2 and the liquid-passing ports 7) in a plan view.

For example, a lid member (not illustrated) covering all of the sample chambers 2 and the liquid-passing ports 7 may be detachably attached to the frame wall portion 855 in order to prevent vaporization of samples. For example, the frame wall portion 855 may serve as a grip for fixing the device 801 at the time of loading of samples.

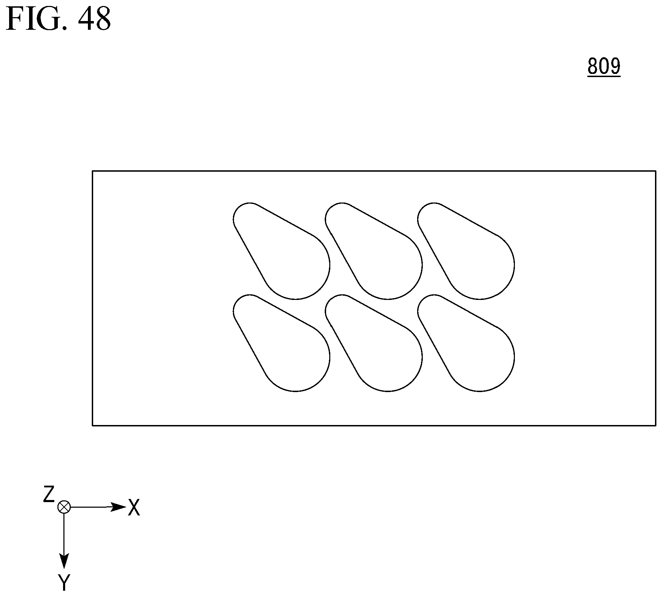

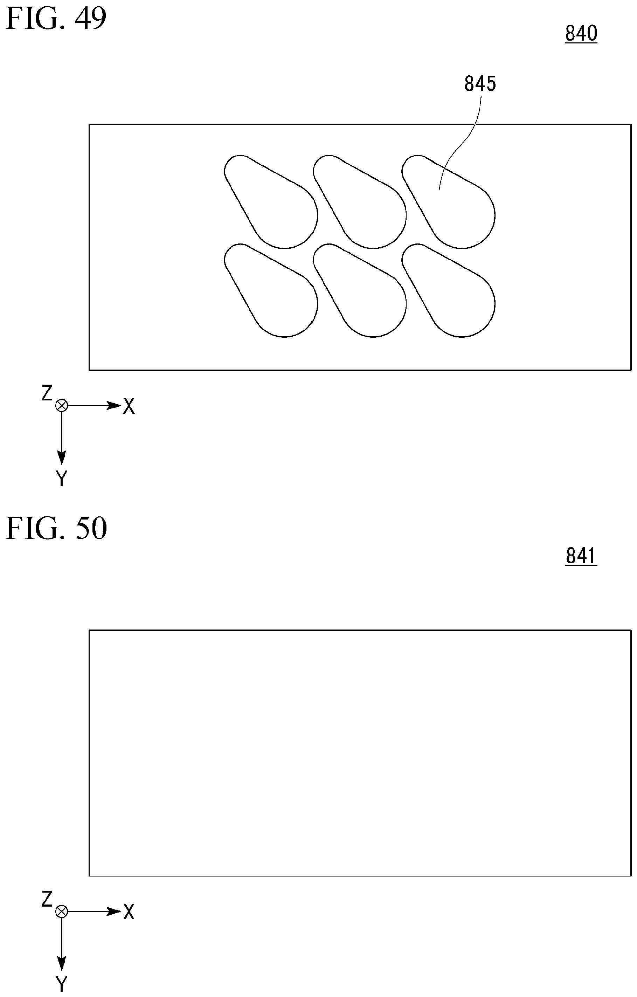

FIG. 48 is a bottom view of the flow passage forming member 809 according to the sixth embodiment. FIG. 49 is a bottom view of an adhesive layer 840 constituting the flow passage forming member 809 according to the sixth embodiment. FIG. 50 is a bottom view of a sheet 841 constituting the flow passage forming member 809 according to the sixth embodiment.

Referring to FIGS. 48 to 50 together, the flow passage forming member 809 has a rectangular shape along the outer shape of the base 850 in a plan view. The flow passage forming member 809 is a structure for allowing a liquid to flow between the sample chamber 2, the accommodation layer 5, and the liquid-passing port 7. The flow passage forming member 809 is for preventing a liquid from flowing between the sample chambers 2 and making the sample chambers 2 independent. The flow passage forming member 809 may include the adhesive layer 840 including a communication hole 845 connecting the liquid-passing port 7 and the sample chamber 2 and the sheet 841 that is attached to the base 850 via the adhesive layer 840.

For example, the adhesive layer 840 may be a double-sided tape (an adhesive sheet including an adhesive layer on both sides) from which the form of the flow passage connecting the liquid-passing port 7 and the sample chamber 2 has been cut out. The adhesive layer 840 is provided on the bottom surface of the base 850 to adhere to the sheet 841. The adhesive layer 840 may be provided on the whole bottom surface of the base 850 except the flow passage connecting the liquid-passing port 7 and the sample chamber 2.

The adhesive layer 840 is not limited to the double-sided tape and may be provided by applying a liquid adhesive such as an epoxy system or an acryl system using an arbitrary method. For example, when the surfaces of the base 850 and the sheet 841 can adhere to each other through direct chemical and physical reforming, both may be caused to adhere using this method.

The material of the adhesive layer 840 can be arbitrarily selected according to a subject accommodated in the sample chambers 2. Examples of the adhesive layer 840 include adhesive sheets using acryl-based, silicon-based, and rubber-based adhesives. The adhesive layer 840 can additionally include one or more materials selected from a group consisting of a material not having cytotoxicity, a material in which cytotoxic components are not eluted, a material having low autofluorescence, a material which is not significantly degraded by gamma irradiation, a material which is not significantly degraded by ethylene oxide steam exposure, a material which is not significantly degraded by ultraviolet irradiation, a material which is not significantly degraded by sterilization treatment, a material which does not dissolve in alcohol, and/or mixtures thereof and preferably includes a material which does not have cytotoxicity and in which cytotoxic components are not eluted.

When the adhesive layer 840 incudes components which are eluted in alcohol or the like and the components can be removed without generating a precipitate by adding water to alcohol including an eluate, the adhesive layer 840 can be used. For example, the device according to the present embodiment may be used by replacing ethanol with water after pre-wetting treatment with ethanol has been performed on the whole device. For example, when an eluate based on ethanol is present in the majority of tapes but particles not passing through the flow passage are extracted or a precipitate not dissolving in water is not attached to the device, the tapes may be used.

For example, the sheet 841 is preferably transparent in a wavelength range from blue to infrared which is used for excitation of a fluorescence microscope. For example, the sheet 841 can additionally include one or more materials selected from a group consisting of a material which does emit fluorescence, a material which is not colored, clouded, or degenerated even through gamma irradiation, a material which is not colored, clouded, or degenerated even through ultraviolet irradiation, a material which is not colored, clouded, or degenerated even through ethylene oxide stem exposure, a material which is not colored, clouded, or degenerated even through sterilization treatment, and/or mixtures thereof and preferably includes a cycloolefin copolymer (COC) or a cycloolefin polymer (COP).

For example, a thickness C1 of the flow passage forming member 809 is equal to or less than 2 mm. For example, in order to enable high-magnification observation using an inverted microscope (an example of a microscope), the thickness C1 of the flow passage forming member 809 is preferably equal to or less than 1.5 mm, more preferably equal to or less than 1.0 mm, and still more preferably equal to or less than 0.5 mm. The thickness C1 of the flow passage forming member 809 corresponds to a distance from the bottom surface of the device 801 to the accommodation layer 5 (see FIG. 43).

The sheet 841 constituting the flow passage forming member 809 may be provided as one film (a single-layered member) or may be provided as a stacked member of a plurality of films. For example, the configuration of the sheet 841 (the number of films) constituting the flow passage forming member 809 can be changed according to design specifications.

For example, the thicknesses of the sheet 841 and the adhesive layer 840 are arbitrary as long as the total thickness of the flow passage forming member 809 is the thickness C1 (for example, equal to or less than 2 mm). For example, the thickness of the sheet 841 is about 188 $\mu$m. For example, the thickness of the adhesive layer 840 is about 50 $\mu$m.

A focal position of a high-magnification objective lens of a microscope is designed in consideration of a medium (for example, air, water, or oil) between a lens and a cover glass and the thickness of glass. **In** the configuration of the device according to the present embodiment, light reaches an observation subject via a lens, a medium to the bottom of the device, water filling a flow passage, and the accommodation portion. Water has a lower refractive index than glass. Accordingly, when the height of the flow passage is high and the layer of water is thick, an optical aberration increases and high-magnification observation becomes difficult. For this prevention, the height of the flow passage is preferably low as long as a flow of a liquid is not hindered. The height of the flow passage is determined by the thickness of the adhesive layer 840. The thickness of the adhesive layer 840 is preferably equal too r less than 100 $\mu$m and more preferably equal to or less than 50 $\mu$m in order to reduce an influence of water in the flow passage of the aberration. A lower limit of the thickness of the adhesive layer 840 is not limited as long as a flow of a liquid is not hindered and is preferably equal to or greater than 20 $\mu$m.

The thickness of the sheet 841 is not limited, but when the thickness is too small, there is a likelihood that the sheet 841 may be deformed upward (to the accommodation layer side) due to a negative pressure generated at the time of suction of a liquid and cells may depart outward from the accommodation layer. The sheet 841 preferably has a thickness in which deformation due to the negative pressure at the time of suction of a liquid decreases. In view of an optical aberration, the sheet 841 is preferably formed of a material of which the refractive index is close to that of glass, and the thickness thereof is preferably equal to or greater than 170 $\mu$m and equal to or less than 200 $\mu$m which is close to that of commercially available cover glass. From the aforementioned description, a COC or a COP with a thickness of about 188 $\mu$m can be preferably used as the sheet 841.

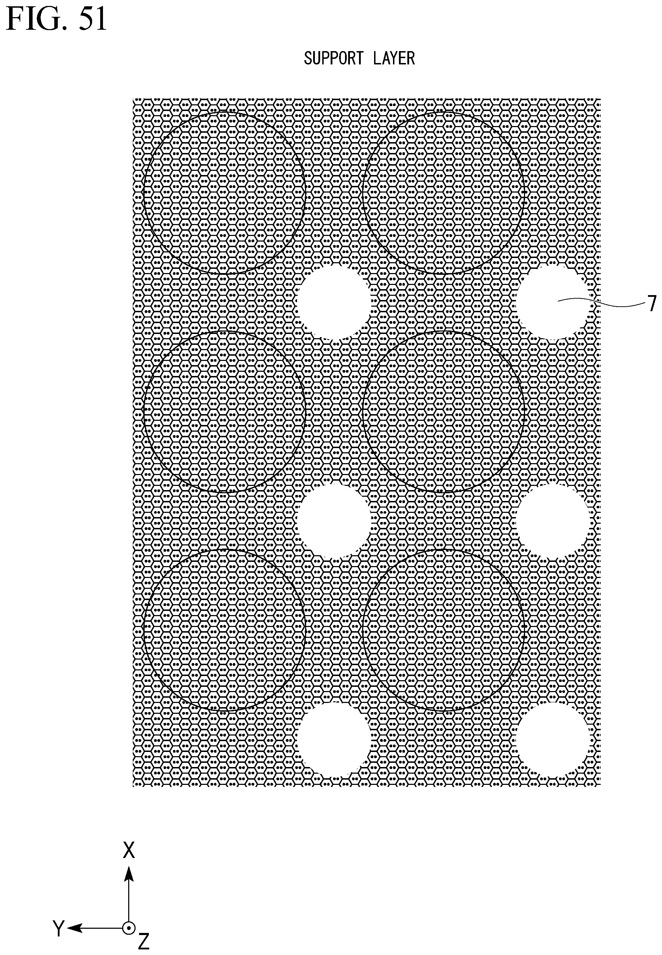

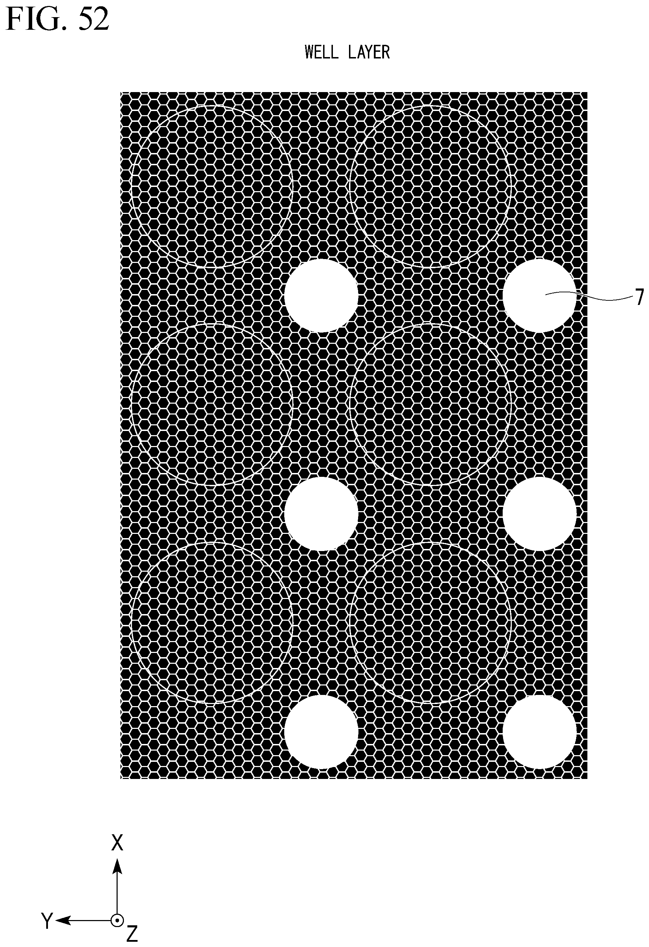

FIG. 51 is a diagram illustrating an example (a support layer) of a reticle pattern of the accommodation layer according to the sixth embodiment. FIG. 52 is a diagram illustrating an example (a well layer) of a reticle pattern of the accommodation layer according to the sixth embodiment. The reticle pattern is a pattern when the accommodation layer is formed of a negative resist.

Referring to FIGS. 51 and 52 together, the accommodation layer 5 may include a support layer and/or a well layer including a pattern (a reticle pattern) which is manufactured using a reticle (an example of a photomask).

In FIGS. 51 and 52, parts surrounded by black circles correspond to openings of the sample chambers 2. The inside of the part surrounded by each black circle may be a through-hole. The outside of the part surrounded by the black circle may not include a through-hole. For example, a film corresponding to the support layer and/or the well layer may be attached to the body 808 using an adhesive.

The accommodation layer 5 is a film-like member including the accommodation portion 4 corresponding to each sample chamber 2. Referring to FIG. 10 together, the accommodation layer 5 may include a well opening 30 of a size through which a sample (a cell or a particle) is able to pass. The accommodation layer 5 may include a well bottom 31 corresponding to the well opening 30. The well bottom 31 includes a through-hole 32 allowing only a liquid to pass therethrough.

**In** the illustrated example, the shape in a plan view of the well opening 30 is hexagonal and may be any shape in a plan view as long as it has a size through which a sample (a cell or a particle) is able to pass. **In** the illustrated example, two through-holes 32 which are circular in a plan view are provided at the center of the well bottom 31, and the shape in a plan view of the through-hole 32 and the number of through-holes 32 are not limited as long as it can allow only a liquid to pass therethrough.

The material of the accommodation layer 5 can be arbitrarily selected according to a subject accommodated in the sample chambers 2. The accommodation layer 5 may additionally include one or more materials selected from a group consisting of a negative photoresist, polyethylene glycol or polydimethyl siloxane (PDMS) having a polymerizable functional group, and/or mixtures thereof and preferably includes a negative photoresist.

Referring to FIGS. 51 and 52 together, white parts (corresponding to liquid-passing ports) in the support layer and the well layer have no resist and correspond to parts in which a hole is formed. For example, wells may be provided on the whole surface other than the liquid-passing ports. Accordingly, wet-spreadability of an adhesive may be controlled.

For example, through-holes may be provided in the bottom of wells in only the parts corresponding to the sample chambers 2. That is, through-holes may not be provided in parts other than the sample chambers 2. Accordingly, it is possible to prevent the accommodation layer 5 from adhering to parts other than the body 808 (an adhesion target member) and necessary parts.

As described above, the device 801 according to the present embodiment is constituted by one component which is the body 808 including the sample chambers 2 and the liquid-passing ports 7.

With this configuration, in comparison with a case in which the device is constituted by a component including the sample chambers 2 and a component including the liquid-passing ports 7, it is possible to reduce the number of components. By decreasing the flow passage height, it is possible to reduce an influence on an optical aberration at the time of high-magnification observation and also to reduce the number of constituent components.

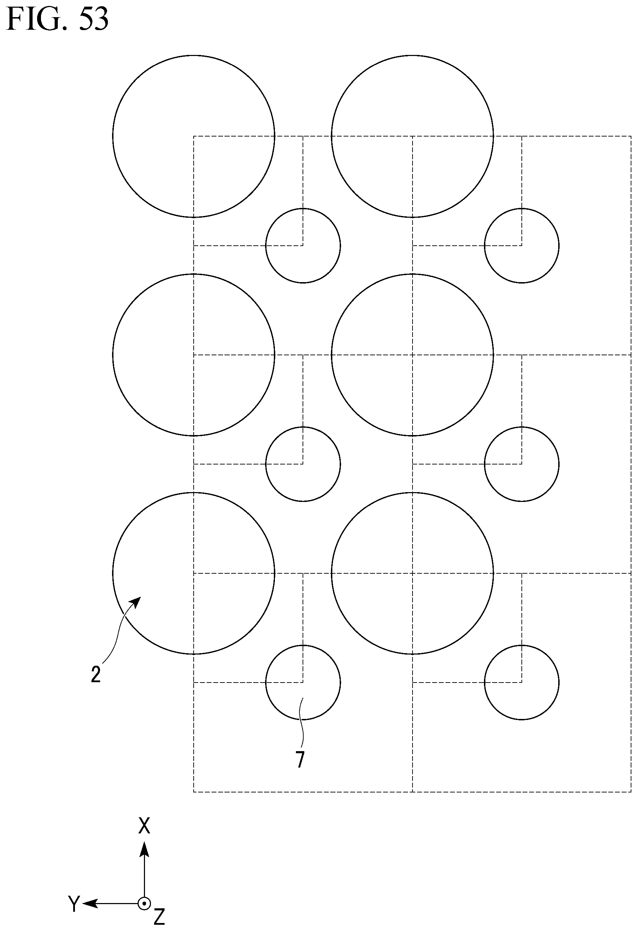

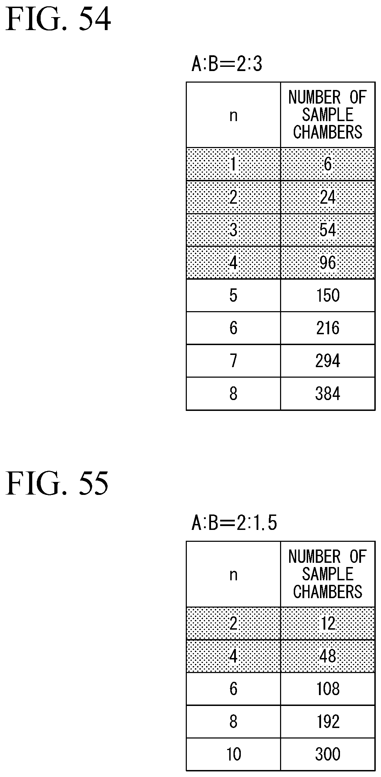

FIG. 53 is a diagram illustrating an example of an arrangement of sample chambers and liquid-passing ports in a microplate. FIG. 54 is a diagram illustrating an example of the number of sample chambers when the ratio A:B of the number sample chambers A arranged in the Y direction to the number of sample chambers B arranged in the X direction in FIG. 53 is 2:3. FIG. 55 is a diagram illustrating an example of the number of sample chambers when the ratio A:B of the number sample chambers A arranged in the Y direction to the number of sample chambers B arranged in the X direction in FIG. 53 is 2:1.5.

Referring to FIGS. 53 to 55 together, the device may have the same rectangular outer shape as a microplate in a plan view. For example, the sample chambers and the liquid-passing ports may be provided such that they are arranged obliquely with respect to the X direction and the Y direction (to form a pair). For example, the liquid-passing ports may be arranged at positions of a half position of pitches between the sample chambers. Accordingly, it is possible to arrange the same number of sample chambers and liquid-passing ports in the microplate.

In FIGS. 54 and 55, the number of sample chambers is calculated using Expression (1).

$$\text{Number of sample chambers} = A \times B \times n2 \qquad \ldots (1)$$

For example, in consideration of liquid passability and operability, hatched ranges in FIGS. 54 and 55 can be preferably applied to the device.

On the other hand, the constituents in the aforementioned embodiments can be replaced with known constituents without departing from the gist of the present invention. The aforementioned modified examples may be combined.

INDUSTRIAL APPLICABILITY

With the device, the observation method, the recovery method, the culture method, and the device manufacturing method according to the present invention, it is possible to independently observe a plurality of samples (cells or particles) in one device.

REFERENCE SIGNS LIST

1 Device
2 Sample chamber
3 Unit
4 Accommodation portion
5 Accommodation layer
6 Structure
7 Liquid-passing port
8 Liquid-passing member
9 Flow passage forming member
10 First member
20 Second member
30 Well opening
31 Well bottom
32 Through-hole
40 Adhesive layer
41 First sheet
42 Second sheet
43 Third sheet
44 Fourth sheet
45 Communication hole
46 Opening hole
C1 Thickness of flow passage forming member

**Claims**

1. A device comprising a plurality of units of which each includes a sample chamber that is able to individually accommodate cells or particles,

   wherein each unit includes:

      an accommodation layer including an accommodation portion corresponding to the sample chamber; and
      a structure allowing a liquid to flow into the accommodation portion, and

   wherein the plurality of units are independent such that there is no flow of the liquid therebetween.

2. The device according to claim 1, wherein the structure includes:

      a liquid-passing member including a liquid-passing port for allowing the liquid to flow to the accommodation portion and having the accommodation layer attached thereto; and
      a flow passage forming member attached to the liquid-passing member with the accommodation layer interposed therebetween and allowing the liquid to flow between the sample chamber, the accommodation layer, and the liquid-passing port.

3. The device according to claim 2, wherein the liquid-passing member includes:

      a first member including the sample chamber and having the accommodation layer attached thereto; and
      a second member including the liquid-passing port and assembled into the first member.

4. The device according to claim 2 or 3, wherein the flow passage forming member includes:

      a first sheet that is transparent; and

a second sheet that is stacked on the first sheet and includes a communication hole connecting the liquid-passing port to the sample chamber.

5. The device according to claim 2 or 3, wherein the flow passage forming member includes:

a first sheet that is transparent;
a second sheet that is stacked on the first sheet and includes a communication hole connecting the liquid-passing port to the sample chamber; and
a third sheet that is stacked on the second sheet and includes an opening hole at positions of the liquid-passing member corresponding to the liquid-passing port and the sample chamber.

6. The device according to claim 2 or 3, wherein the flow passage forming member includes:

a first sheet that is transparent;
a second sheet that is stacked on the first sheet and includes a communication hole connecting the liquid-passing port to the sample chamber;
a third sheet that is stacked on the second sheet and includes an opening hole at positions of the liquid-passing member corresponding to the liquid-passing port and the sample chamber; and
a fourth sheet that is stacked on the third sheet and includes the same shape in a plan view as the third sheet, and

wherein an adhesive layer is provided on both sides of the second sheet and both sides of the fourth sheet.

7. The device according to claim 2 or 3, wherein the thickness of the flow passage forming member is equal to or less than 2 mm.

8. The device according to claim 1 or 2, wherein the accommodation layer includes:

a well array filter including a well opening of a size through which the cell or the particle is able to pass; and
a well bottom corresponding to the well opening, and

wherein the well bottom includes a through-hole through which only the liquid is able to pass.

9. The device according to claim 1 or 2, wherein the accommodation layer is formed of a negative resist.

10. The device according to claim 1 or 2, wherein the device has an outer shape of the same size as slide glass in a plan view, and
wherein two, four, six, or eight sample chambers are provided in the device.

11. The device according to claim 1 or 2, wherein the device has the same rectangular outer shape as a microplate in a plan view,

wherein A sample chambers are provided in a short-side direction of the device and B sample chambers are provided in a long-side direction of the device, and
wherein a ratio A:B of the number of sample chambers A arranged in the short-side direction in the device to the number of sample chambers B arranged in the long-side direction is 2:3, 2:1.5, or 2:1.

12. The device according to claim 1 or 2, wherein the device has an outer shape of the same size as a microplate in a plan view, and
wherein 32 sample chambers or 48 sample chambers are provided in the device.

13. An observation method of individually observing the cells or the particles using the device according to claim 1 or 2 and a microscope.

14. A recovery method of individually recovering the cells or the particles using the device according to claim 1 or 2.

15. A culture method of culturing the cells using the device according to claim 1 or 2.

16. A device manufacturing method of manufacturing the device according to claim 1 or 2, the device manufacturing method comprising:

a first step of attaching the accommodation layer to a liquid-passing member including a liquid-passing port for allowing the liquid to flow to the accommodation portion; and
a second step of attaching a flow passage forming member for allowing the liquid to flow between the sample chamber, the accommodation layer, and the liquid-passing port to the liquid-passing member with the accommodation layer interposed therebetween after the first step.

17. The device manufacturing method of manufacturing the device according to claim 16, wherein the first step includes

an attachment step of attaching the accommodation layer to a first member including the sample chamber and an assembly step of manufacturing the liquid-passing member by assembling a second member including the liquid-passing port to the first member after the attachment step.

FIG. 1

<u>1</u>

FIG. 2

FIG. 3

EP 4 621 041 A1

FIG. 4

# FIG. 5

<u>10</u>

VI

VI

2  11  12  2  11  12  2  11  12  13

X
Y  Z

# FIG. 6

2  11  12  2  11  12  2  11  12

10

13

39

14  4  14  4  14  4  5

Z
Y  X

FIG. 7

FIG. 8

FIG. 9

# FIG. 10

4(5)

FIG. 11

20

XV
24   7   21   24   7   21   21   7   24   22
XII                                    XII
25

XIV                                    XIV

24   7        24   7        24   7
XV

X
Y ← ⊙ Z

EP 4 621 041 A1

FIG. 12

20

25

22

26    23    21    21    21    26

Z
Y ← ⊗ X

FIG. 13

2    12    2    12    2    12    25

20

22

26    23    13    21    21    21    26

10

Z
Y ← ⊗ X

FIG. 14

FIG. 15

FIG. 16

FIG. 17

20

FIG. 18

## FIG. 19

## FIG. 20

FIG. 21

<u>9</u>

40
44
40
43
40
42
40
41

Z
Y X

FIG. 22

42

7
45
45

2
45

7

X
Y Z

FIG. 23

FIG. 24

FIG. 25

<u>303</u>

# FIG. 26

303

FIG. 27

FIG. 28

303

FIG. 29

401

2

2

2

2

2

2

Y

Z X

FIG. 30

501

EP 4 621 041 A1

FIG. 31

EP 4 621 041 A1

FIG. 32

605A

11   11   11

Y ← ⊗ Z
↓
X

FIG. 33

605B

11

Y ← ⊗ Z
↓
X

FIG. 34

605C

11

Y ← ⊗ Z
↓
X

FIG. 35

FIG. 36

FIG. 37

FIG. 38

FIG. 39

EP 4 621 041 A1

FIG. 40

EP 4 621 041 A1

FIG. 41

SAMPLE CHAMBER 1   LIQUID-PASSING PORT 1   SAMPLE CHAMBER 2   LIQUID-PASSING PORT 2

EP 4 621 041 A1

# FIG. 42

EP 4 621 041 A1

FIG. 43

# FIG. 44

808

Y
Z X

# FIG. 45

808

Z X

Y

FIG. 46

EP 4 621 041 A1

FIG. 47

850

852

851

853

FIG. 48

809

FIG. 49

840

845

Z ⊗ →X

Y

FIG. 50

841

Z ⊗ →X

Y

# FIG. 51

SUPPORT LAYER

## FIG. 52

WELL LAYER

## FIG. 53

## FIG. 54

A:B=2:3

| n | NUMBER OF SAMPLE CHAMBERS |
|---|---|
| 1 | 6 |
| 2 | 24 |
| 3 | 54 |
| 4 | 96 |
| 5 | 150 |
| 6 | 216 |
| 7 | 294 |
| 8 | 384 |

## FIG. 55

A:B=2:1.5

| n | NUMBER OF SAMPLE CHAMBERS |
|---|---|
| 2 | 12 |
| 4 | 48 |
| 6 | 108 |
| 8 | 192 |
| 10 | 300 |

## INTERNATIONAL SEARCH REPORT

| | International application No. |
|---|---|
| | **PCT/JP2023/040557** |

| A. | CLASSIFICATION OF SUBJECT MATTER |
|---|---|

*C12M 1/34*(2006.01)i; *C12M 1/00*(2006.01)i; *C12M 3/00*(2006.01)i; *C12N 1/00*(2006.01)i; *C12N 1/02*(2006.01)i; *C12Q 1/02*(2006.01)i; *G01N 37/00*(2006.01)i

FI: C12M1/34 A; C12M1/00 C; C12M3/00 Z; C12N1/00 A; C12N1/02; C12Q1/02; G01N37/00 101

According to International Patent Classification (IPC) or to both national classification and IPC

| B. | FIELDS SEARCHED |
|---|---|

Minimum documentation searched (classification system followed by classification symbols)

C12M1/00-3/10; C12N1/00-7/08; C12Q1/00-3/00; G01N37/00; B01D53/22;61/00-71/82;C02F1/44; B01D39/00-41/04; B01L1/00-99/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | JP 2019-146548 A (NATIONAL UNIVERSITY CORPORATION YOKOHAMA NATIONAL UNIVERSITY) 05 September 2019 (2019-09-05) claims, paragraphs [0012], [0016], [0029]-[0037], [0076], fig. 1-3 | 1, 10-15 |
| Y | claims, paragraphs [0012], [0016], [0029]-[0037], [0076], fig. 1-3 | 8-9 |
| A | claims, paragraphs [0012], [0016], [0029]-[0037], [0076], fig. 1-3 | 2-7, 16-17 |
| Y | WO 2021/065771 A1 (TOKYO OHKA KOGYO CO., LTD.) 08 April 2021 (2021-04-08) paragraphs [0030]-[0032] | 8-9 |
| X | JP 2020-10683 A (TOKYO WOMEN'S MEDICAL COLLEGE) 23 January 2020 (2020-01-23) claims, paragraphs [0023], [0028], [0030], [0053], fig. 1-3 | 1-3, 7, 10-17 |
| Y | claims, paragraphs [0023], [0028], [0030], [0053], fig. 1-3 | 8-9 |
| A | claims, paragraphs [0023], [0028], [0030], [0053], fig. 1-3 | 4-6 |

☐ Further documents are listed in the continuation of Box C.    ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **17 January 2024** | **30 January 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

**INTERNATIONAL SEARCH REPORT**
Information on patent family members

International application No.

**PCT/JP2023/040557**

| Patent document cited in search report | | | Publication date (day/month/year) | Patent family member(s) | | | Publication date (day/month/year) |
|---|---|---|---|---|---|---|---|
| JP | 2019-146548 | A | 05 September 2019 | WO | 2019/167951 | A1 | |
| WO | 2021/065771 | A1 | 08 April 2021 | US paragraphs [0039]-[0041] | 2022/0297127 | A1 | |
| JP | 2020-10683 | A | 23 January 2020 | (Family: none) | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2022181803 A
- WO 2021065771 A